# EUROPEAN PATENT SPECIFICATION

(11) **EP 3 833 366 B1**
(45) Date of publication and mention of the grant of the patent: **03.12.2025**
(21) Application number: 19846452.1
(22) Date of filing: 08.08.2019
(51) Int. Cl.: A61K 31/22, A61K 35/66, A61K 36/064, A61K 35/744, A23L 33/14, A23C 9/13, A23C 9/127, A23C 9/12, A61P 25/28, A61P 31/04, A61P 29/00, A61K 31/215, A61K 31/137, A61K 31/192, A61K 31/05, A61K 31/404, A61K 35/747, A61K 35/741

(54) **MICROORGANISM MIXTURES, MOLECULES DERIVED THEREFROM, AND METHODS OF USE THEREOF**
MIKROORGANISMENGEMISCHE, DAVON ABGELEITETE MOLEKÜLE UND VERWENDUNGSVERFAHREN DAFÜR
MÉLANGES DE MICRO-ORGANISMES, MOLÉCULES DÉRIVÉES DE CEUX-CI ET LEURS PROCÉDÉS D'UTILISATION

(30) Priority: 08.08.2018 US 201862715875 P
(43) Date of publication of application: 16.06.2021
(73) Proprietor: B.G. Negev Technologies and Applications Ltd., at Ben-Gurion University, 84105 Beer-Sheva (IL)
(72) Inventor: JELINEK, Raz, 71799 Reut (IL); MALKA, Orit, 8448408 Beer Sheva (IL); KALSON, Dorin, 8483506 Beer Sheva (IL); MEIJLER, Michael, 8496500 Omer (IL); KUSHMARO, Ariel, 7031719 Beer Yaakov (IL)
(74) Representative: Pearl Cohen Zedek Latzer Baratz UK LLP
(86) International application number: PCT/IL2019/050903
(87) International publication number: WO 2020/031191

(56) References cited:
- KR-A- 20070 005 937
- KR-A- 20100 062 450
- KR-A- 20110 119 609
- XING-FU CAI ET AL: "Anti-inflammatory Constituents from Solanum nigrum", BULLETIN OF THE KOREAN CHEMICAL SOCIETY, vol. 31, no. 1, 20 January 2010 (2010-01-20), pages 199 - 201, XP055683590, DOI: 10.5012/bkcs.2010.31.01.199
- G�NTERT MATTHIAS ET AL: "HRGC und HRGC-MS angewandt auf Weinaromastoffe geringerer Fl�chtigkeit", ZEITSCHRIFT F�R LEBENSMITTEL-UNTERSUCHUNG UND -FORSCHUNG, vol. 182, no. 3, 1 March 1986 (1986-03-01), pages 200 - 204, XP055909413, ISSN: 0044-3026, DOI: 10.1007/BF01042128
- MALKA ORIT ET AL: "Tryptophol Acetate and Tyrosol Acetate, Small-Molecule Metabolites Identified in a Probiotic Mixture, Inhibit Hyperinflammation", JOURNAL OF INNATE IMMUNITY, vol. 15, no. 1, 22 December 2023 (2023-12-22), CH, pages 531 - 547, XP093135285, ISSN: 1662-811X, DOI: 10.1159/000529782
- XING-FU CAI , YOUNG-WON CHIN , SEI-RYAN OH , OK-KYUNG KWON , KYUNG-SEOP AHN , HYEONG-KYU LEE: "Anti-inflammatory constituents from Solanum nigrum", BULLETIN OF THE KOREAN CHEMICAL SOCIETY, vol. 31, no. 1, 20 January 2010 (2010-01-20), pages 199 - 201, XP055683590, DOI: 10.5012/bkcs.2010.31.01.199
- PATRICIA ARACLI BOLLA , PAULA CARASI . MARIA DE LOS ANGELES SERRADELL , GRACIELA LILIANA DE ANTONI: "Kefir-isolated Lactococcus lactis subsp . lactis inhibits the cytotoxic effect of Clostridium difficile in vitro", vol. 80, no. 1, 10 December 2012 (2012-12-10), pages 96 - 102, XP055683594, Retrieved from the Internet <URL:https://www.cambridge.org/core/journals/journal-of-dairy-research/article/kefirisolated-lactococcus-lactis-subsp-lactis-inhibits-the-cytotoxic-effect-of-clostridium-difficile-in-vitro/579FE02B0A97BC7F46406F52DFB36EBB> [retrieved on 20191127]
- ROMANIN D E , LLOPIS S , GENOVES S , MARTORELL P , RAMON V D , GARROTE G L , RUMBO M : "Probiotic yeast Kluyveromyces marxianus CIDCA 8154 shows anti- inflammatory and anti-oxidative stress properties in in vivo models", BENEFICIAL MICROBES, vol. 7, no. 1, 13 November 2015 (2015-11-13), pages 83 - 93, XP009526211, ISSN: 1876-2883, DOI: 10.3920/BM2015.0066

## Description

### CROSS REFERENCE TO RELATED APPLICATION

This application claims the benefit of priority of U.S. Provisional Patent Application No. 62/715,875 titled "MICROORGANISM MIXTURES, MOLECULES DERIVED THEREFROM, AND METHODS OF USE THEREOF", filed August 8, 2018.

### FIELD OF INVENTION

The present invention is in the field of microbiology.

### BACKGROUND OF THE INVENTION

In light of the spread of bacterial resistance to existing antibiotics, there is a constant and urgent need for novel antibacterial agents, operating through alternative bacterial killing or inhibition mechanisms. Promising strategies have recently focused on interfering bacterial communication pathways, also known as "quorum sensing" (QS). QS bacteria release chemical molecules termed "autoinducers" which in many instances affect gene expression in neighboring bacterial cells, contributing to population modulation through bacteria-bacteria signaling. Both Gram-positive and Gram-negative bacteria use quorum sensing communication circuits to regulate varied physiological activities. Processes modulated through quorum sensing include virulence, competence, conjugation, antibiotic production, motility, and biofilm formation. In general, Gram-negative bacteria use acylated homoserine lactones (AHLs) as autoinducers, and Gram-positive bacteria use processed oligo-peptides (DPD). Additionally, data suggesting that bacterial autoinducers also activate specific biological responses of the host organism is mounting.

Identification and use of substances that interfere and/or disrupt in quorum sensing pathways of pathogenic bacteria and thus block their virulence, is of high importance. Related approaches aim at developing compounds which activate communication pathways of bacteria exhibiting beneficial health properties. Inhibition of QS by analogous molecules may provide an alternative to conventional antibiotics, with a significant advantage of being less prone to resistance development.

Xing-fu Cai et al. (Bulletin of the Korean Chemical Society, vol. 31, no. 1, 20.01.2010, pages 199-201) disclose anti-inflammatory constituents from solanum nigrum. A 50% ethanolic extract of Solanum nigrum contained inter alia caffeic acid, i.e. a 4-ethylphenol derivative, and tryptophol acetate, i.e. a tryptophol derivative, but these two compounds were found to be inactive.

### SUMMARY OF THE INVENTION

The present invention is directed to a composition comprising derivatives of Tryptophol and/or 4-Ethyl-Phenol, such as for reducing biofilm production. Also disclosed is a microorganism mixture comprising the yeast Kluyveromyces marxianus and at least one probiotic microorganism and use thereof.

According to the invention, the Tryptophol derivative is Tryptophol acetate, and the 4-Ethyl-Phenol derivative is Tyrosol acetate. Compositions comprising only Tryptophol derivatives other than Tryptophol acetate and/or only 4-Ethyl-Phenol derivatives other than Tyrosol acetate are not part of the invention, and are referred to only for comparison or reference.

Any reference to a method of treatment of the human or animal body by therapy involving a certain compound or composition is to be understood as referring to said compound or composition for use in said method.

According to one aspect, there is provided a composition comprising: (1) a Tryptophol derivative; and (2) 4-Ethyl-Phenol derivative; and at least one pharmaceutically acceptable carrier, wherein the Tryptophol derivative and the 4-Ethyl-Phenol derivative are in a ratio of 10:1 - 1:10 w/w ratio.

According to another aspect, there is provided a microorganism mixture comprising: (1) K. marxianus; and (2) at least one probiotic microorganism, wherein the microorganism mixture comprises at least 3% K. marxianus.

According to another aspect, there is provided a method for treating a disease selected from the group consisting of: an inflammatory disease, an infectious disease, and an amyloid aggregates-related disease, in a subject in need thereof, the method comprising: administering to the subject a therapeutically effective amount of a pharmaceutical composition comprising any one of: at least one molecule selected from a Tryptophol derivative and a 4-Ethyl-Phenol derivative, and the herein disclosed microorganism mixture.

In some embodiments, the Tryptophol derivative and 4-Ethyl-Phenol derivative are each present at a concentration of at least 1 µM within the composition.

In some embodiments, the Tryptophol derivative is at a concentration of at least 0.1 µM within the composition.

In some embodiments, the Tryptophol derivative and the 4-Ethyl-Phenol derivative are in w/w ratio ranging from 2:1 (w/w) - 1:2 (w/w).

In some embodiments, the composition further comprises Kluyveromyces marxianus; and at least one probiotic microorganism.

In some embodiments, the composition is for use in any one of reducing microbial activity, treating an inflammatory disease, and an amyloid aggregates-related disease.

In some embodiments, the probiotic microorganism is a probiotic bacterium.

In some embodiments, the probiotic bacterium is selected form the group consisting of: Lactobacillus, Propionibacterium, Lactococcus, and Leuconostoc.

In some embodiments, the mixture is suspended in a medium.

In some embodiments, the medium is milk.

In some embodiments, the mixture is kefir.

In some embodiments, the mixture further comprises a Tryptophol derivative, a 4-Ethyl-Phenol derivative, or a combination thereof.

In some embodiments, the Tryptophol derivative and the 4-Ethyl-Phenol derivative are produced by K. marxianus.

In some embodiments, the microorganism mixture is for use in foodstuff.

In some embodiments, the microorganism mixture is for use in any one of reducing microbial activity, treating an inflammatory disease, and an amyloid aggregates-related disease.

In some embodiments, the infectious disease comprises a load of a microorganism, a biofilm derived therefrom, or both.

In some embodiments, the microorganism is selected from the group consisting of: a virus, a fungus, a parasite, a yeast, a bacterium, and a protozoon.

In some embodiments, the fungus belongs to a genus selected from the group consisting of: Botrytis, Penicillium and Sclerotinia.

In some embodiments, the bacterium belongs to a genus selected form the group consisting of: Vibrio, Salmonella, Staphylococcus, and Pseudomonas.

In some embodiments, the composition has a half maximal inhibitory concentration (IC50) of 0.1-500 µM.

In some embodiments, the subject is afflicted with at least one disease selected from the group consisting of: an inflammatory disease, an infectious disease, and an amyloid-related disease.

In some embodiments, the inflammatory disease is an inflammatory bowel disease.

In some embodiments, the inflammatory bowel disease is ulcerative colitis or Crohn's disease.

In some embodiments, the amyloid aggregates -related disease is a neurodegenerative disease.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Further embodiments and the full scope of applicability of the present invention will become apparent from the detailed description given hereinafter. However, it should be understood that the detailed description and specific examples, while indicating preferred embodiments of the invention, are given by way of illustration.

### BRIEF DESCRIPTION OF THE DRAWINGS

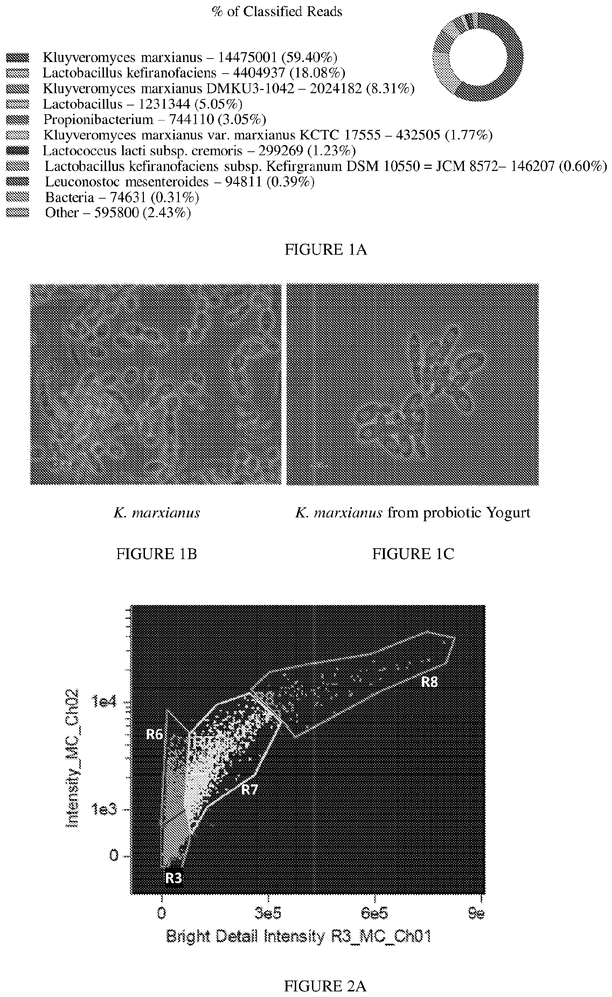

**Figs. 1A-1C** are identification of microorganism mixture in a probiotic yogurt. (**1A**) is distribution pie chart of microorganisms in a probiotic yogurt based on a BLAST comparison in the One Codex data platform for applied microbial genomics. (**1B**) and (**1C**) are light microscope images of a Kluyveromyces marxianus in a monoculture and from a probiotic yogurt, respectively.
**Figs. 2A-2E** describe the portions of microorganism subpopulations in a probiotic yogurt. (**2A**) is a scatterplot of auto fluorescence intensity vs. BF detail intensity of population R5 (19,539 events). Distinct subpopulations R3, R6, and R7 are visible including additional composite subpopulation R8. (**2B**-**2E**) are micrographs showing cells of populations R3, R6, R7 and R8, respectively.

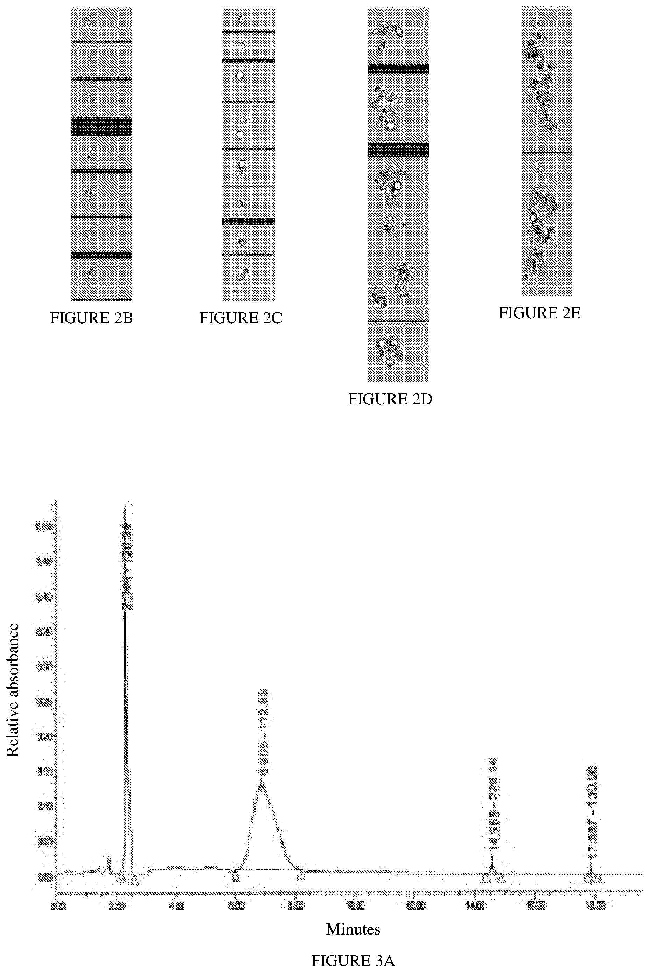

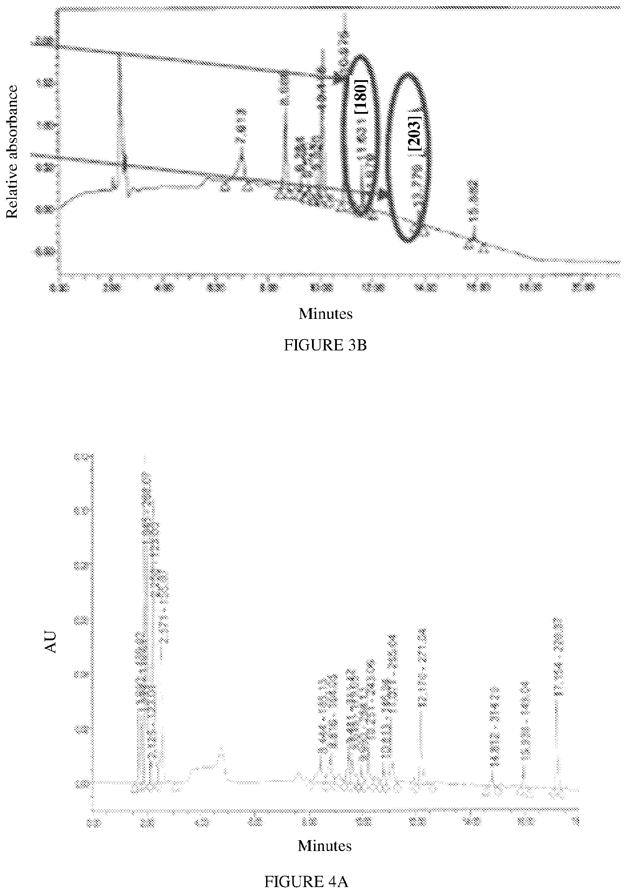

**Figs. 3A-3B** are liquid chromatography-mass spectrometry (LC-MS) chromatograms of (**3A**) milk extract and (**3B**) probiotic yogurt. Two molecules, corresponding to masses 180 and 203 (hereinafter referred to as the "180" molecule and the "203" molecule) are present only in the probiotic yogurt and are encircled.
**Figs. 4A-4B** are LC-MS chromatograms of (**4A**) Kluyveromyces marxianus culture medium and (**4B**) Kluyveromyces marxianus crude. The 180 and 203 molecules are present only in the Kluyveromyces marxianus crude and are encircled.

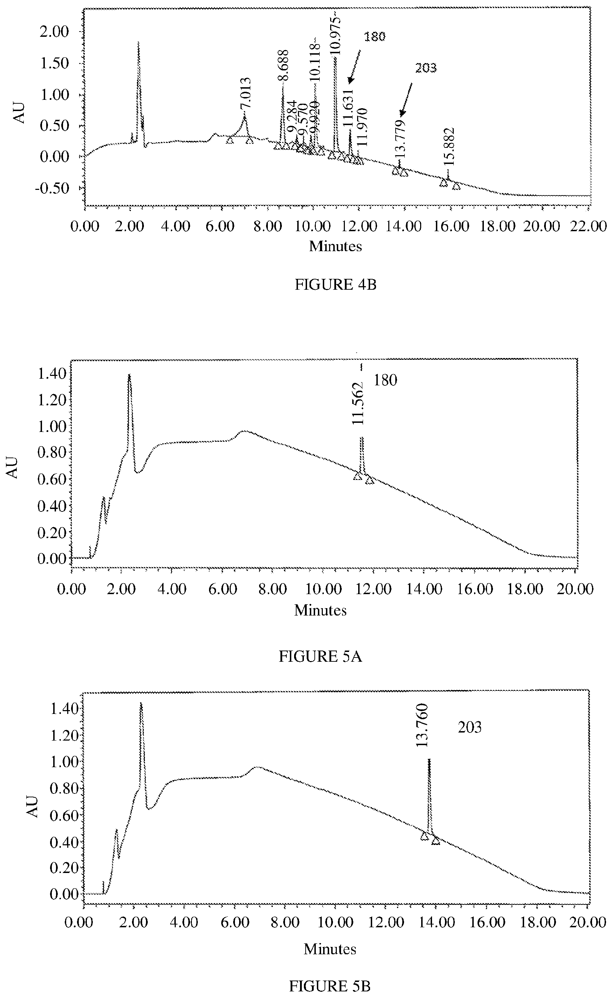

**Figs. 5A-5B** are LC-MS chromatograms of Kluyveromyces marxianus crude extract fractions after separation (**5A**) the 180 molecule and (**5B**) the 203 molecule.

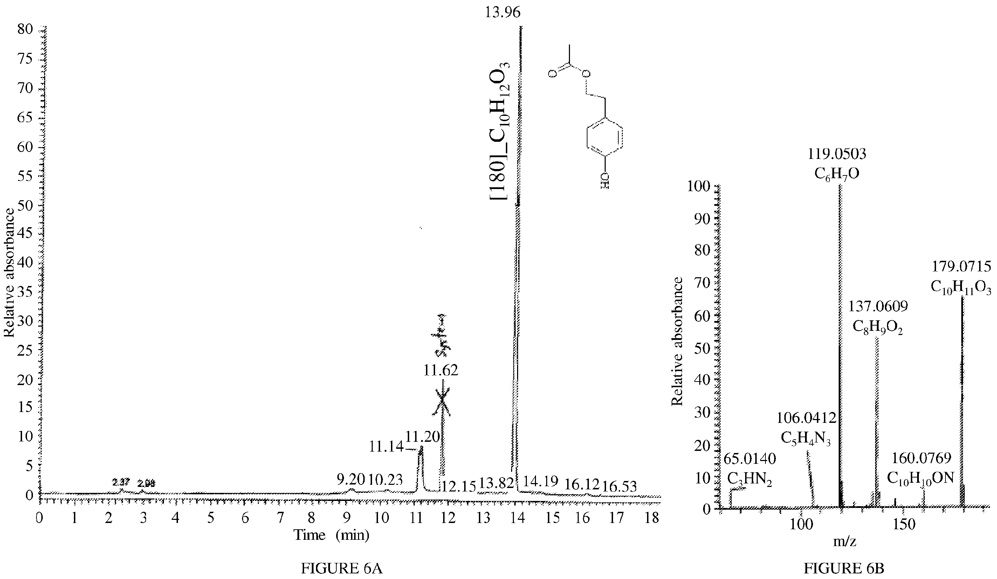

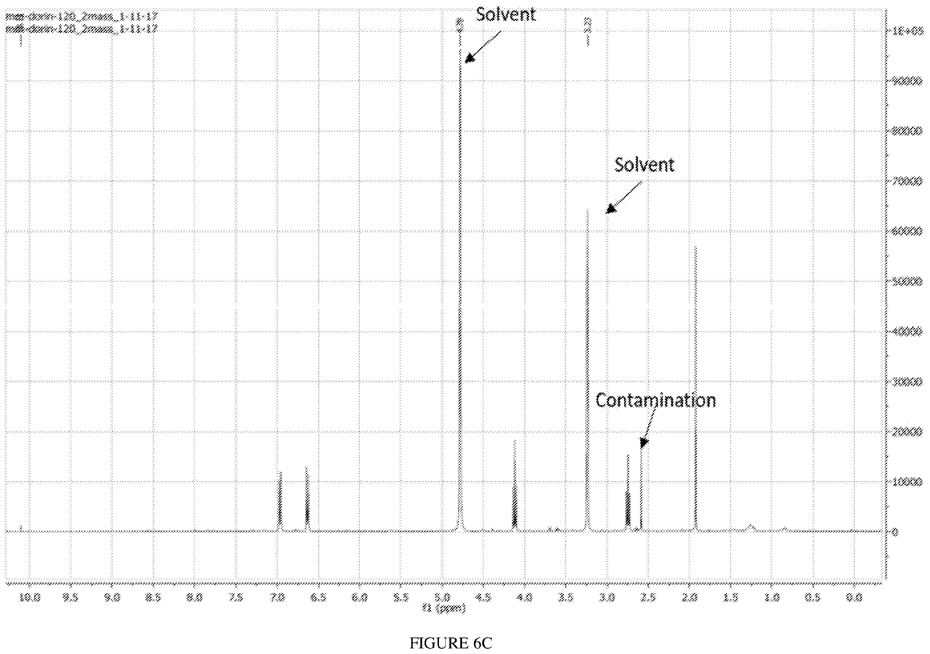

**Figs. 6A-6C** show weight and structural analysis of synthetic Tyrosol acetate. (**6A and 6B**) are LC-MS (orbitrap) chromatograms of the molecule having a mass of 180. (**6A**) is the retention time spectra and (**6B**) is the m/z spectra. (**6C**) is 1H NMR spectrum of Tyrosol acetate with peak distribution: δ 2.04 (3H, s), 2.87 (2H, t, J = 7.0 Hz), 4.28 (2H, t, J = 7.0 Hz), 6.70 (2H, ddd, J = 8.2, 2.4, 0.5 Hz), 6.95 (2H, ddd, J = 8.2, 1.0, 0.5 Hz).

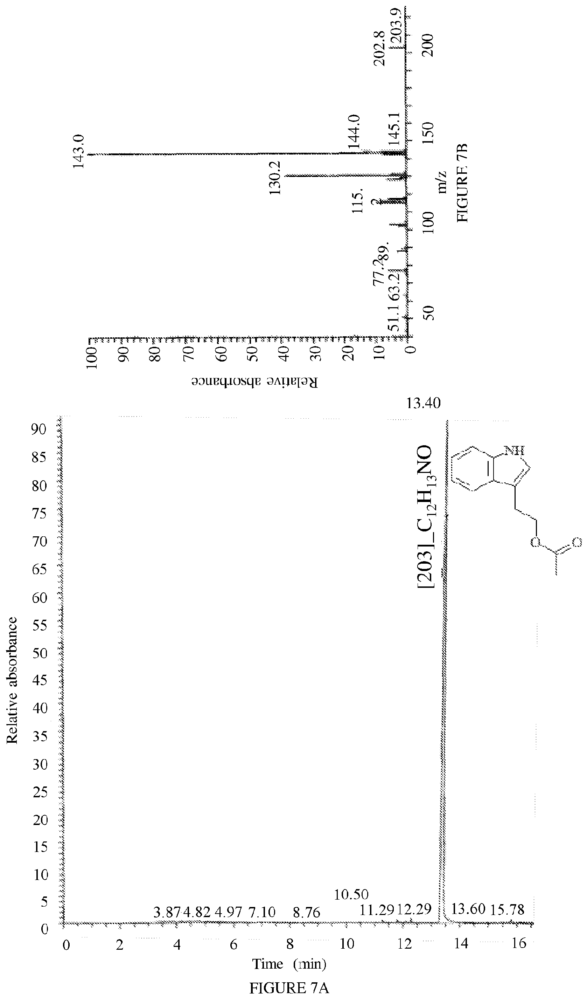

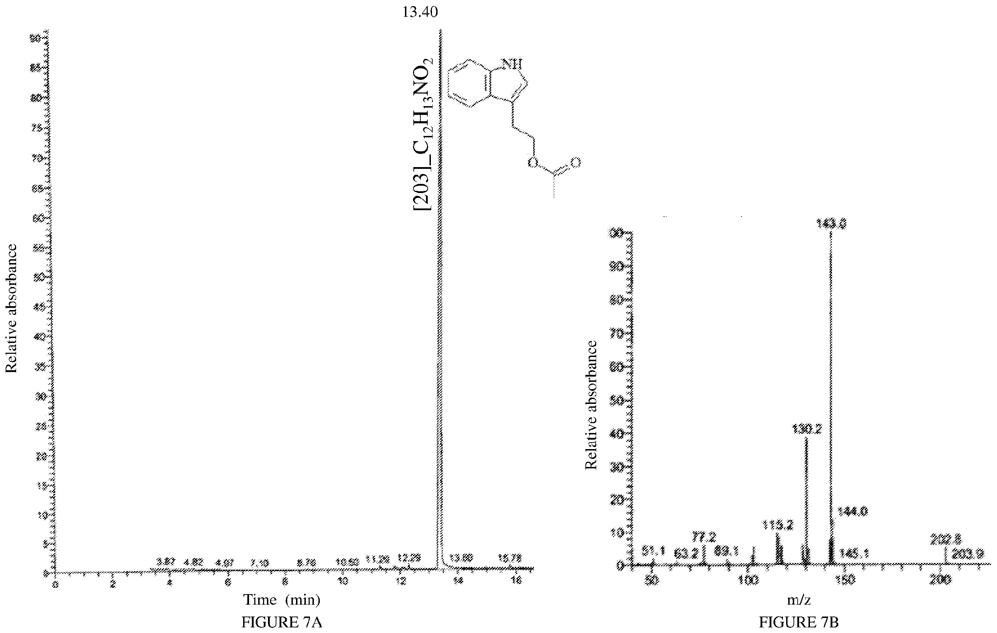

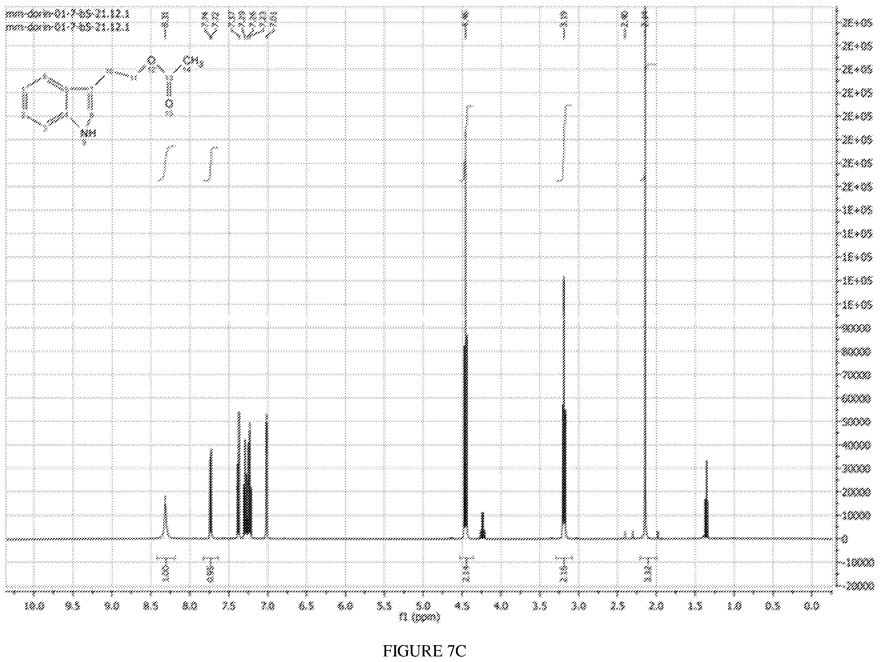

**Figs. 7A-7C** show weight and structural analysis of the synthetic Tryptophol acetate. (**7A and 7B**) are LC-MS (orbitrap) chromatograms of the molecule having a mass of 203. (**7A**) is the retention time spectra and (**7B**) is the m/z spectra. (**7C**) is 1H NMR spectrum of Tryptophol acetate with peak distribution: δ 2.05 (3H, s), 3.08 (2H, t, J = 5.2 Hz), 4.40 (2H, t, J = 5.2 Hz), 6.93-7.12 (2H, 6.98 (ddd, J = 8.0, 7.8, 1.2 Hz), 7.07 (ddd, J = 8.0, 7.8, 1.6 Hz)), 7.30-7.36 (2H, 7.33 (dddd, J = 8.0, 1.2, 0.5, 0.5 Hz), 7.32 (t, J = 0.5 Hz)), 7.62 (1H, dddd, J = 8.0, 1.6, 0.5, 0.5 Hz).

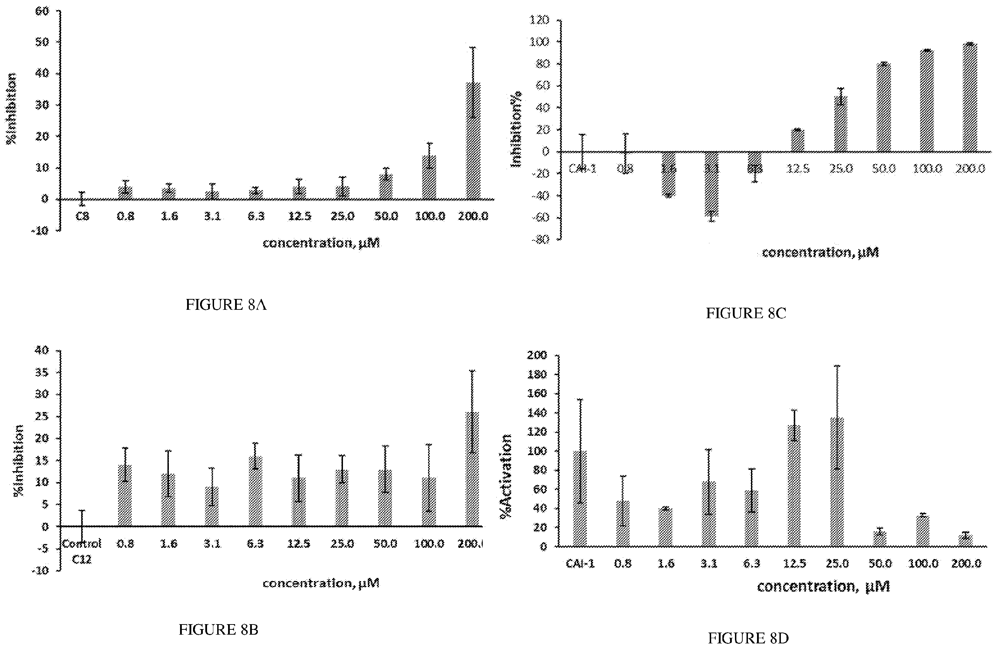

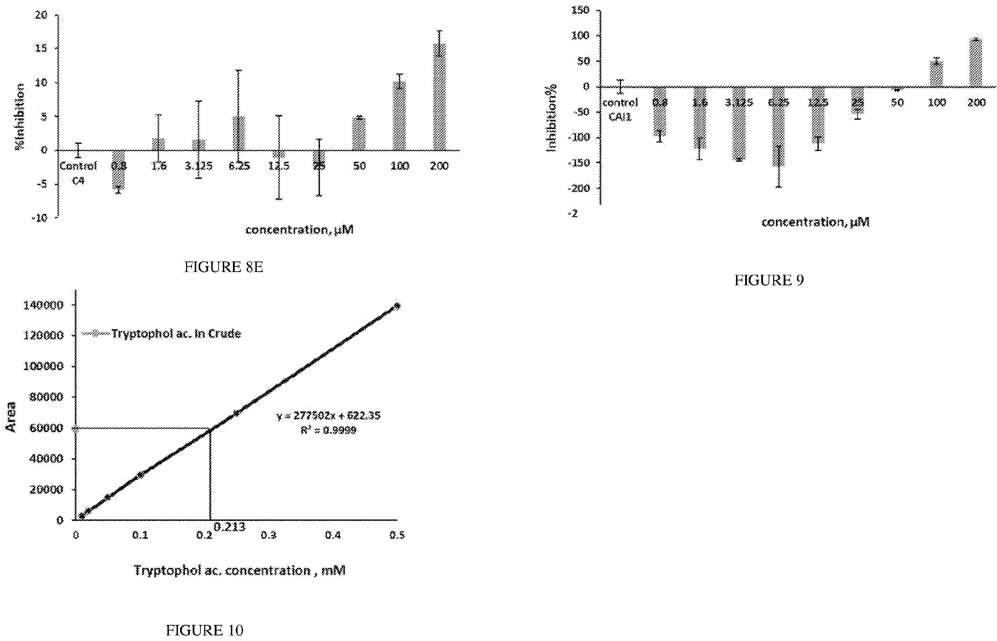

**Figs. 8A-8E** are vertical bar graphs showing bioluminescence screening of quorum sensing (QS) inhibition or activation in the presence of synthetic Tryptophol acetate. The assay was performed using mutant bacteria lacking genes encoding autoinducers. Bacteria were also cloned with a bioluminescence reporter plasmid. N-acyl Homoserine Lactone (AHL) was used for activation (agonist) or inhibition (antagonist) of the following bacteria: Agrobacterium tumefaciens, which responds to the C8 autoinducer (AI), Vibrio cholerae responds to the CAI1 autoinducer, Pseudomonas aeruginosa RhlA responds to the C4 AI and Pseudomonas aeruginosa LasR responds to the C12 AI. (**8A**) shows Tryptophol acetate inhibits QS in A. tumefaciens. (**8B**) shows Tryptophol acetate has a low inhibition effect on QS in P. aeruginosa (LasR). (**8C**-**8D**) show Tryptophol acetate activates QS in low volume and inhibits QS with an IC₅₀ of 12.7 ± 2.0 µM in high volume, in V. cholera. (**8E**) shows Tryptophol acetate has a low inhibition effect on QS in high volume in P. aeruginosa (RhlA).
**Fig. 9** is vertical bar graphs showing Tyrosol acetate activates QS in low volume and totally inhibits QS in high volume with an IC₅₀ of 24.4 ± 2.4 µM in V. cholera.
**Fig. 10** is the calibration curve of the synthetic Tryptophol acetate used to quantify the material secreted in the probiotic yogurt crude extract showing a concentration of 213 µM in probiotic yogurt (measured in a batch of 800 ml).

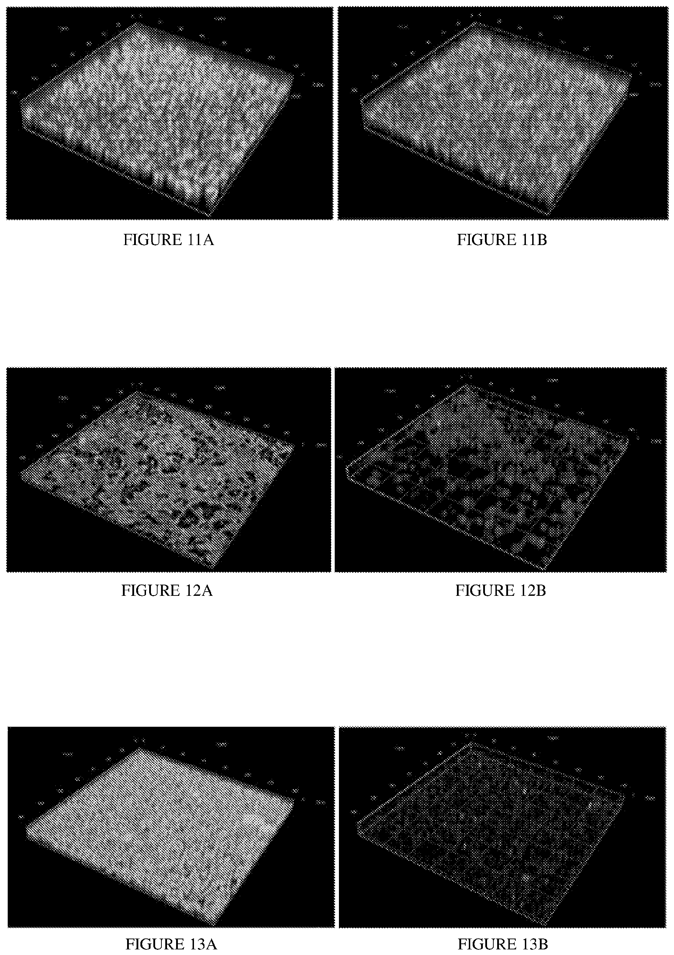

Figs. **11A-11B** are confocal laser scanning microscopy (CLSM) images describing P. aeruginosa in the absence (**11A,** control with 1% DMSO) or presence (**11B,** 20 µM) of Tryptophol acetate. No influence was observed on biofilm formation.
**Figs. 12A-12B** are CLSM images describing Salmonella in the absence (**12A,** control with 1% DMSO) or presence (**12B**, 50 µM) of Tryptophol acetate. A significant influence was observed on biofilm formation.
**Figs. 13A-13B** are CLSM images describing Staphylococcus aureus in the absence (**13A,** control with 1% DMSO) or presence (**13B,** 50 µM) of Tryptophol acetate. A significant influence was observed on biofilm formation.

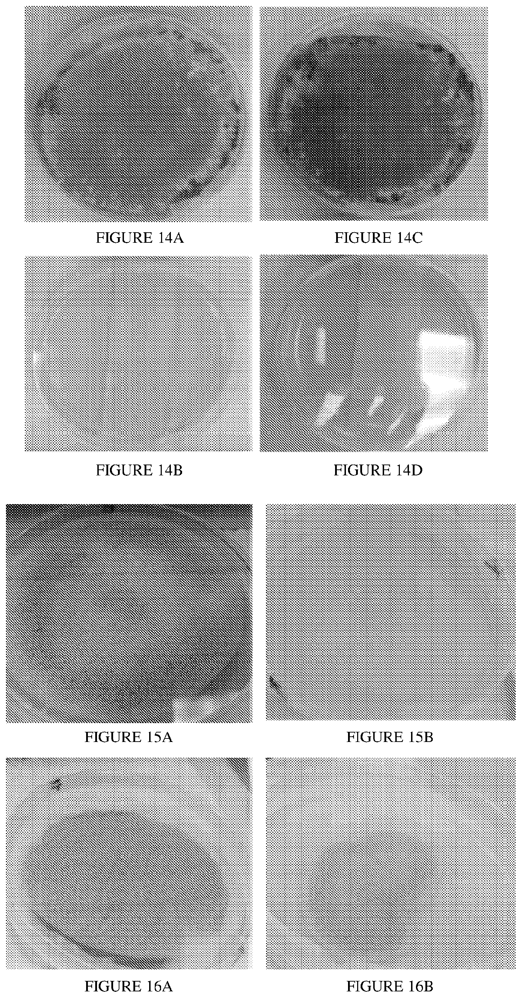

**Figs. 14A-14D** are images describing bioactivity of a probiotic yogurt towards fungal growth. Probiotic yogurt inhibited growth of Sclerotinia sclerotiorum on potato dextrose agar plates for as long as (**14A-14B**) 12 days or (**14C-14D**) 19 days. Inhibited plates comprising probiotic yogurt extract (**14B** and **14D**) and control plates showing S. sclerotium growth (**14A** and **14C**) are presented.
**Figs. 15A-15B** are images describing bioactivity of a probiotic yogurt extract towards fungal growth. (**15A**) Botrytis fungus grown on a control plate; (**15B**) an inhibited Botrytis fungus seeded on a probiotic yogurt extract-supplemented plate documented 22 days post seeding.
**Figs. 16A-16B** are images describing bioactivity of a probiotic yogurt extract towards fungal growth. (**16A**) Penicillium fungus grown on a control plate; (**16B**) an inhibited Penicillium fungus seeded on a probiotic yogurt extract-supplemented plate documented 22 days post seeding.

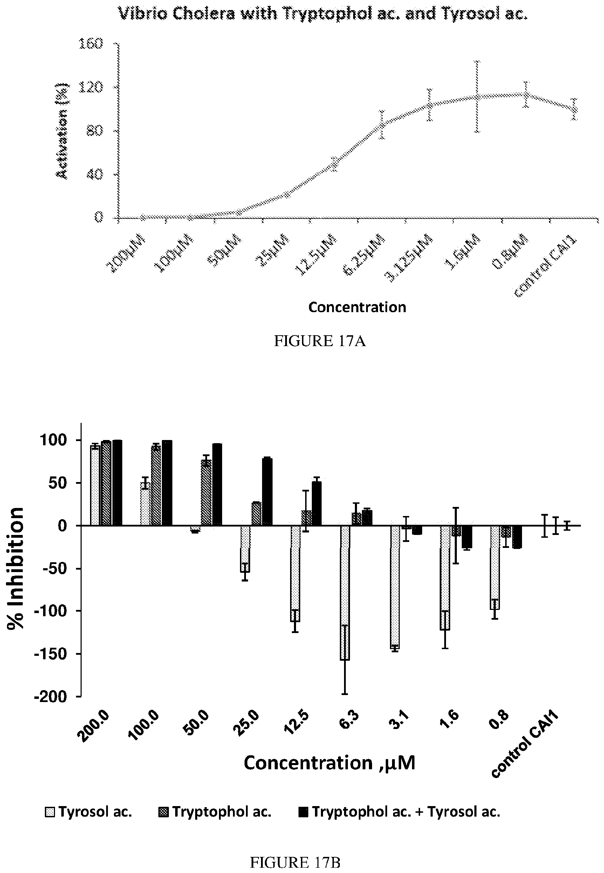

**Figs. 17A-17B** are graphs showing the synergistic effect of Tryptophol acetate and Tyrosol acetate on activation of QS in V. cholera. (**17A**) is a graph showing the effect of a composition of Tryptophol acetate and Tyrosol acetate administered together at a ratio of 1:1, with an IC₅₀ of 11.6 ± 0.9 µM. (**17B**) is a vertical bar graph showing the comparative effect of each of Tryptophol acetate and Tyrosol acetate and a composition of both at the same concentration range as described in (**17A**).

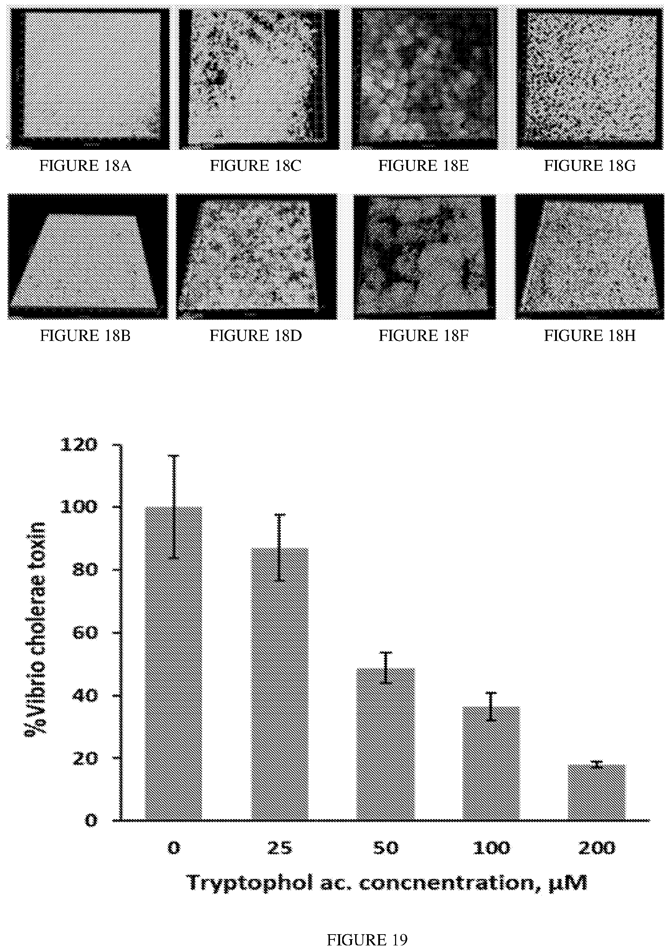

**Figs. 18A-18H** are CLSM images describing biofilm modulation activity of a composition of Tryptophol acetate and Tyrosol acetate examined in a mutated MM920 V. cholerae strain (ΔCqsA ΔluxQ). V. cholerae CqsA mutant biofilms (**18A** and **18E**) are much thicker and denser than the respective biofilms generated upon addition of the autoinducer alone (**18B** and **18F**; which promotes quorum sensing, thus disrupts biofilm formation), Tryptophol acetate and Tyrosol acetate (**18D and 18H**), and particularly the three compounds together (**18C and 18G**) demonstrated a significant synergistic effect in disruption of normal biofilm growth - resulting in a different biofilm morphology. Upper row comprises top images and lower row comprises their corresponding 3D images.
**Fig. 19** is a vertical bar graph showing quantification of V. cholerae toxins in the absence (0, control with 1% (v/v) DMSO) or presence in different concentrations of Tryptophol acetate (25, 50, 100, and 200 µM).

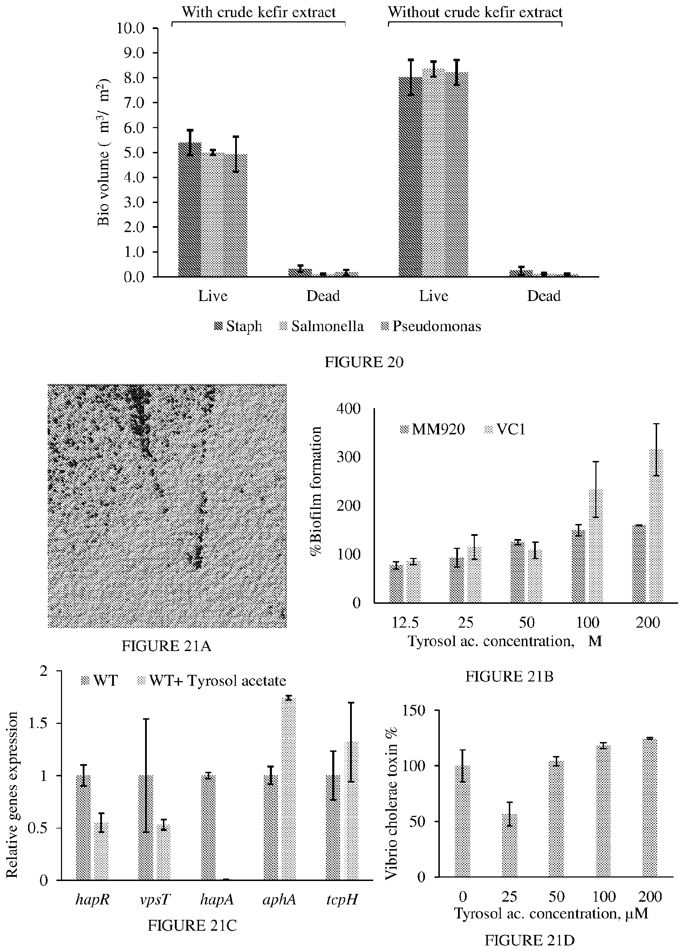

**Fig. 20** is a vertical bar graph showing cell-viability assays in the absence or presence of the crude kefir extract. The results confirmed that the crude kefir extract did not adversely impact bacterial cell growth.
**Figs. 21A-21D** are an image and vertical bar graphs showing the effect of Tyrosol acetate in concentration of 100 µM on Vibrio Cholera. (**21A**) Representative biofilm formation obtained from confocal z-stacks using IMARIS software. Scale bar, 50 µm. (**21B**) Quantitative biofilm volume obtained through Crystal violet assay using VC1 strain (the wild type) and the bioassay strain MM920. (**21C**) Effect of tyrosol acetate on the expression of QS genes in a wild type strain assessed by RT-qPCR. (**21D**) Quantification of Cholerae toxins by GM1-ELISA.

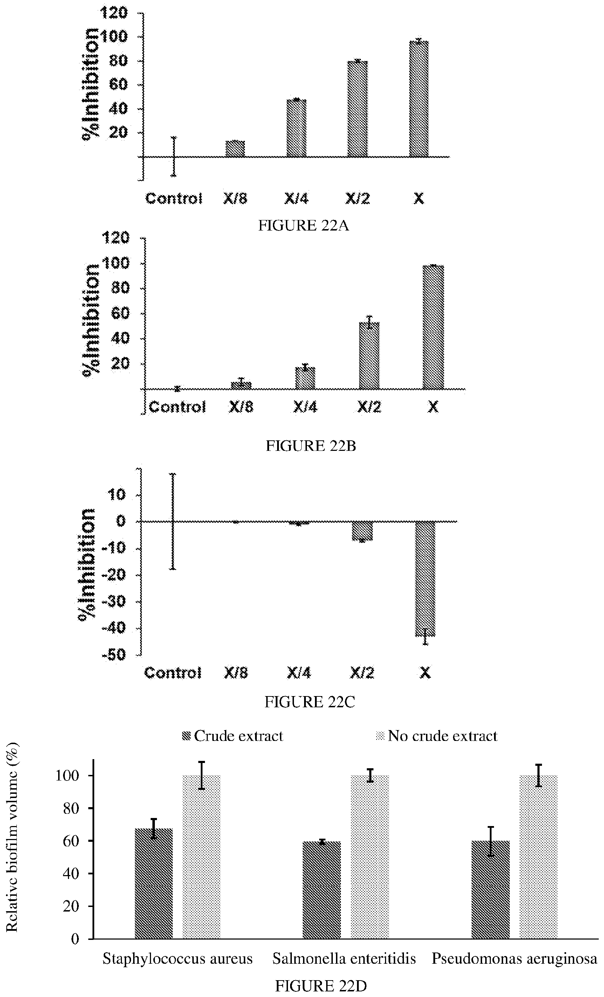

**Figs. 22A**-**22D** are vertical bar graphs showing quorum sensing effects of probiotic yogurt biomass crude extract. (**22A**-**22C**) QS Inhibition/Activation of Vibrio cholerae (**22A**), Agrobacterium tumefaciens (**22B**), and Vibrio harveyi (**22C**) reporter strain bioluminescence by the yogurt biomass crude extract. (**22D**) Static anti-biofilm activity. Graphs show the biofilm volume generated from three different WT bacteria - Staphylococcus aureus, Salmonella enteritidis and Pseudomonas aeruginosa in the absence or presence of the probiotic yogurt (e.g., kefir) crude extract. Data are means ± SD. n = 3. P value: * P<0.12, **P<0.01, ***P<0.002.

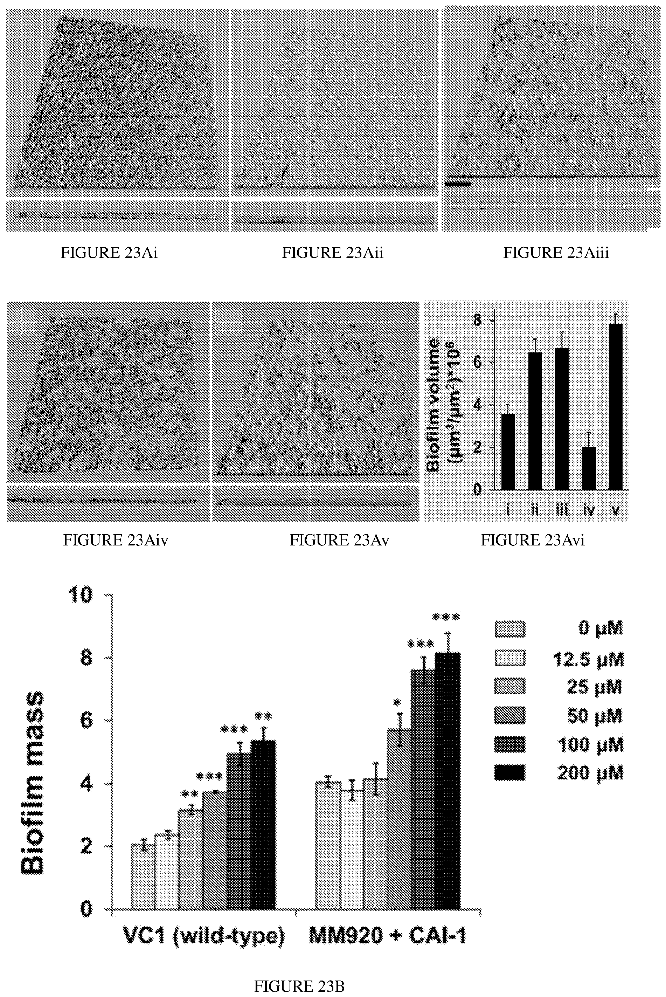

**Figs. 23A-23C** are micrographs and vertical bar graphs showing the effect of Tryptophol acetate on *Vibrio cholerae.* (**23Ai-23Av**) are representative images of biofilm formation obtained from confocal z-stacks using IMARIS software. Scale bar, 50 µm. **(23Ai)** biofilm matrixes of V. *cholerae VC1 (WT);* (**23Aii**) *V. cholerae VC1* in presence of 100 µM tryptophol acetate; (**23Aiii**) *V. cholerae MM920* alone; (**23Aiv**) V. *cholerae MM920* with 900 nM CAI-1; and (**23Av**) *V. cholerae MM920* with 900 nM CAI-1 and in the presence of 100 µM tryptophol acetate. (**23Avi**) a bar graph showing biofilm volume per area quantities corresponding to the biofilm shown in **23Ai-23Av.** (**23B**) a vertical bar graph demonstrating *V. cholerae* quantitative biofilm volume obtained through crystal violet staining at different concentrations of Tryptophol acetate (0, 12.5, 25, 50, 100 and 200 µM) for 24 hr in microtiter plate. Error bars indicate the standard deviations of 4 measurements. * P <0.001, ** P <0.0001, *** P <0.000001 vs. control. (**23C**) A vertical bar graph showing the effect of tryptophol acetate on the expression of QS genes in a wild type strain, assessed by quantitative reverse transcription-polymerase chain reaction (RT-qPCR). The relative magnitude of gene expression levels was defined as the copy number of cDNA of genes in the QS pathway: *hapR, vpsT, hapA, aphA,* and *ctxA,* normalized to a reference housekeeping gene not affected by the treatment. Error bars indicate the standard deviations of 4 measurements. * P <0.001, ** P <0.0001 vs. untreated bacteria.

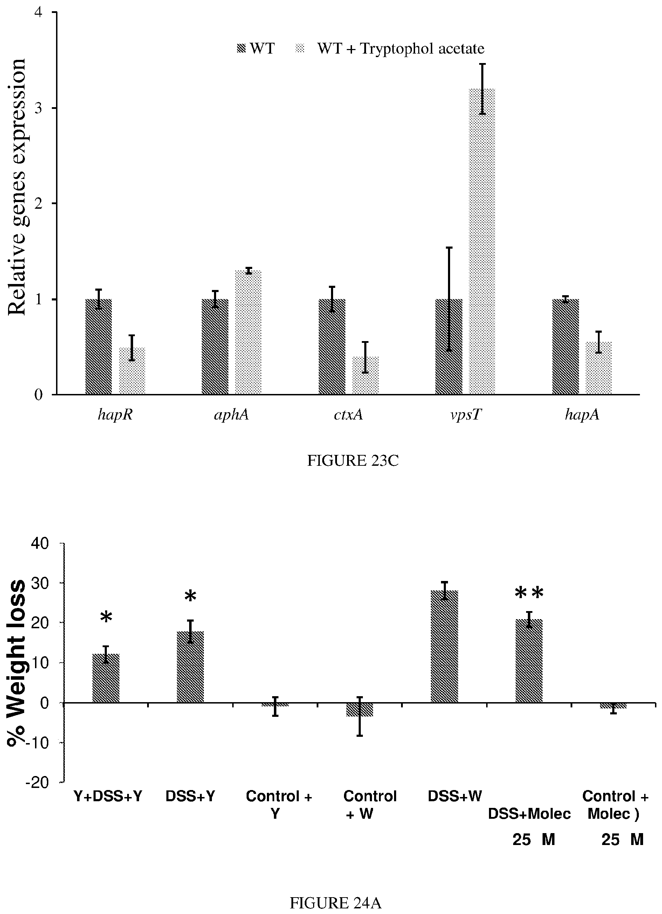

**Figs. 24A-24B** are vertical bar graphs showing that the herein disclosed probiotic yogurt and molecules identified therein reduce weight loss in a murine inflammatory bowel disease (IBD) model. (**24A**) shows experiment repetition number 2, and (**24B**) shows experiment repetition number 3. Weight loss was calculated as the percentage change compared with the body weight before initiation of the experiment. Significant results were observed in body weight loss between Y+DSS+Y; DSS+Y and DSS+Mole (either at 25 µM, or 50 µM) compared to DSS+W. In addition, there was no significant body weight loss effect in the group receiving commercial yogurt (Yc+DSS+Yc or DSS+Yc). Groups: Control + W - Regular food-no Dextran Sodium Sulfate (DSS); DDS + W - Regular food; Control + Y - Testfood (Yogurt)-no DSS; DSS + Y - Testfood (Yogurt); Control + mole - Molecular Test Item (oral, Tyrosol acetate and Tryptophol acetate, final concentration 25 µM or 50 µM)-no DSS; DSS + mole - Molecular Test Item (oral, Tyrosol acetate and Tryptophol acetate, final concentration 25 µM or 50 µM); Control + Yc - Testfood (Commercial Yogurt)-no DSS; DSS + Yc - Testfood (Commercial Yogurt); Y + DSS + Y - Early administration of Yogurt before DSS and after; and Yc + DSS + Yc - Early administration of commercial Yogurt before DSS and after. In (**24A**) * P<0.000003, ** P<0.001. In (24B) * P<0.00005, ** P<0.05.

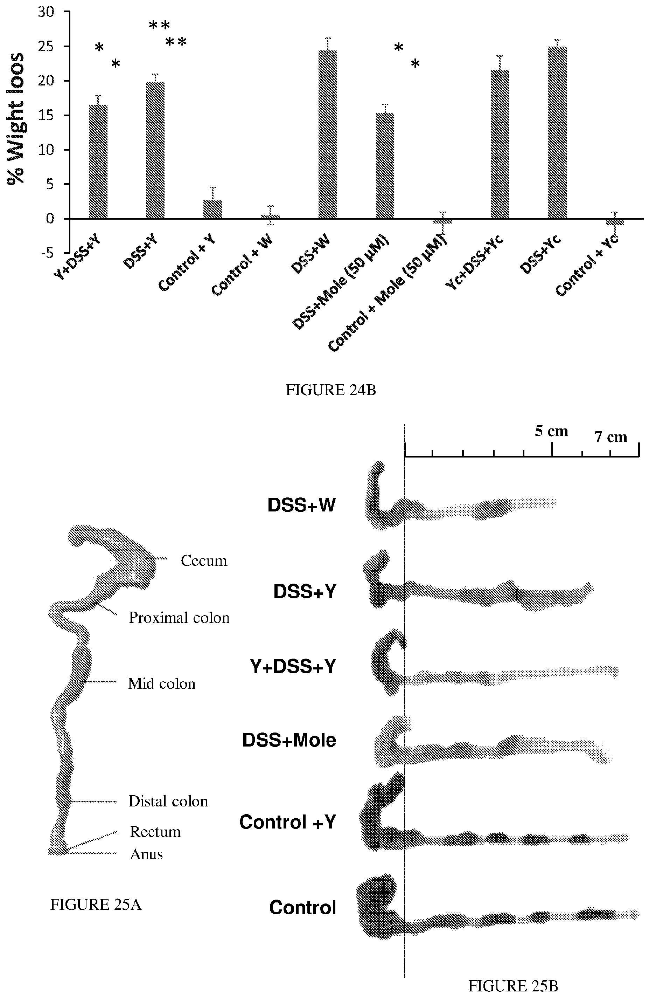

**Figs. 25A**-**25D** are an illustration, images and vertical bar graphs showing that the herein disclosed probiotic yogurt and molecules identified therein reduce colon shortening in a murine IBD model. (**25A**) an illustration of a murine large intestine tract, comprising: Caecum, proximal colon, mid colon, distal colon, rectum and anus. (**25B**) lengths of colons after 7 days of DSS treatment. Images are selected representatives of one out of the 6 experiments performed. (**25C**) shows measurements on day 10 of experiment repetition number 2, and (**25D**) shows measurements on day 10 of experiment repetition number 3. Colon length were measured from the rectum to the caecum at the endpoint of the experiment. Shortening of the colon was observed in mice treated with DSS (DSS+W) compared the colon of mice treated with DSS and fed with the herein disclosed probiotic yogurt before the DSS (Y+DSS+Y). Furthermore, a statistically significant reduced shortening of colon length was observed in mice treated with molecules (50 µM) identified in the herein disclosed probiotic yogurt. * P<0.05. Experimental groups were as in **Fig. 24**.

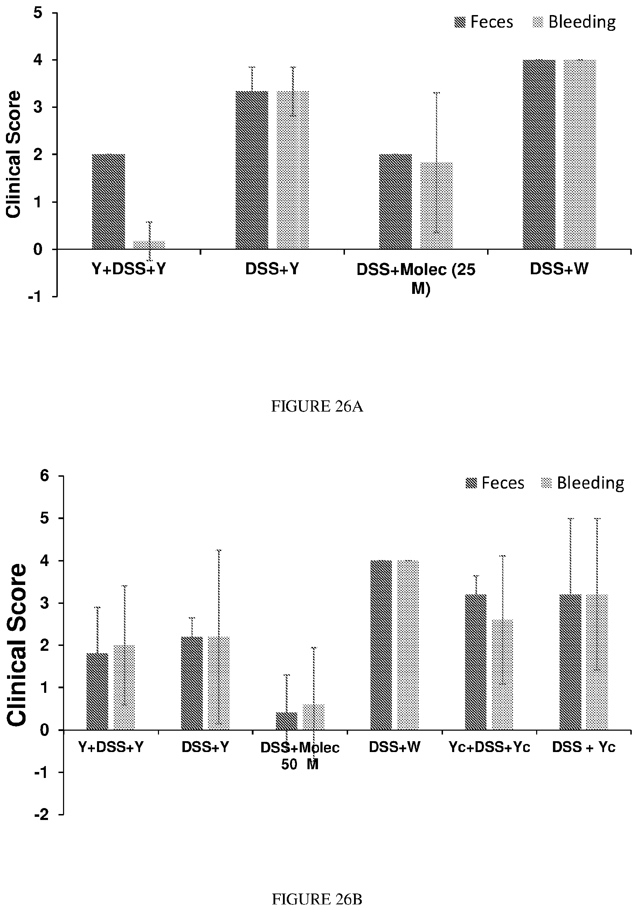

**Figs. 26A-26B** are vertical bar graphs showing the effect of the herein disclosed probiotic yogurt and of molecules identified therein on disease activity index (DAI) determined in experiment repetition 2 (**26A**) and experiment repetition 3 (**26B**). DAI was determined based on measured stool consistency and blood in the stool. Clinical signs were recorded every other day. The results showed significant decrease in the clinical score in mice treated with yogurt before DSS (Y+DSS+Y) in repetition 2 (**26A**) and molecules identified in the herein disclosed probiotic yogurt in concentration of 50 µM (DSS + Molec) in repetition 3 (**26B**). Experimental groups were as in **Figs**. **24-25**
**Figs**. **27A**-**27D** are micrographs of colon histological sections. (**27A**) revealed intensive tissue damage with loss of crypt cells from the gut lining and infiltration of inflammatory cells into the colon in all DSS-treated mice. (**27B**) in colon of mice fed with yogurt before DSS (Y+DSS+Y) tissue structure was preserved with only minimal inflammation and initial signs of colon tissue repair (encircled). (**27C**) section of the colon of mice fed with the herein disclosed probiotic yogurt after DSS (DSS + Y). (**27D**) is control colon (e.g., healthy colon).

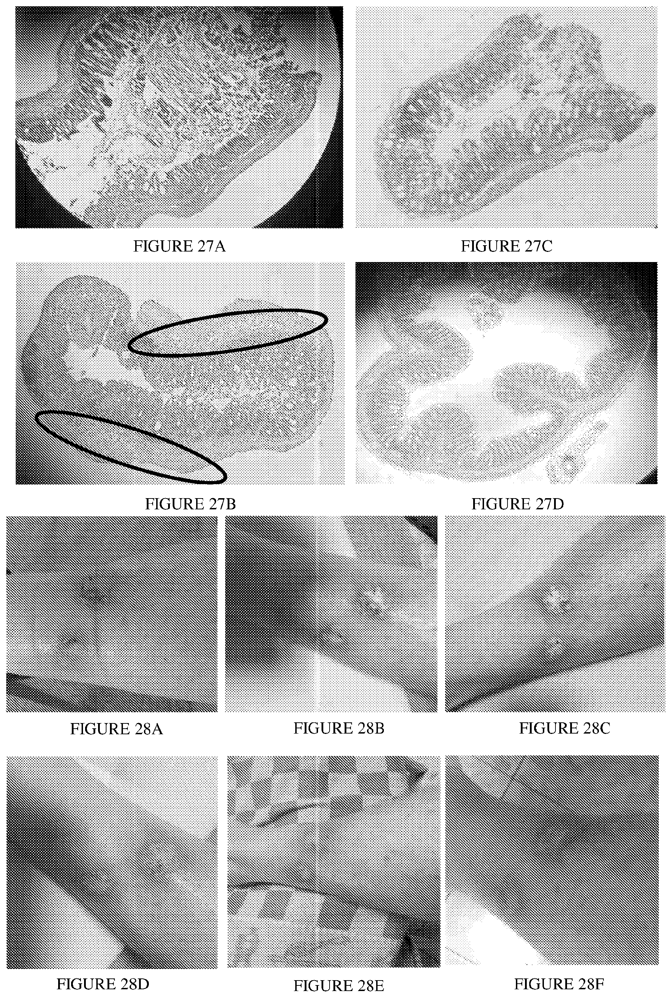

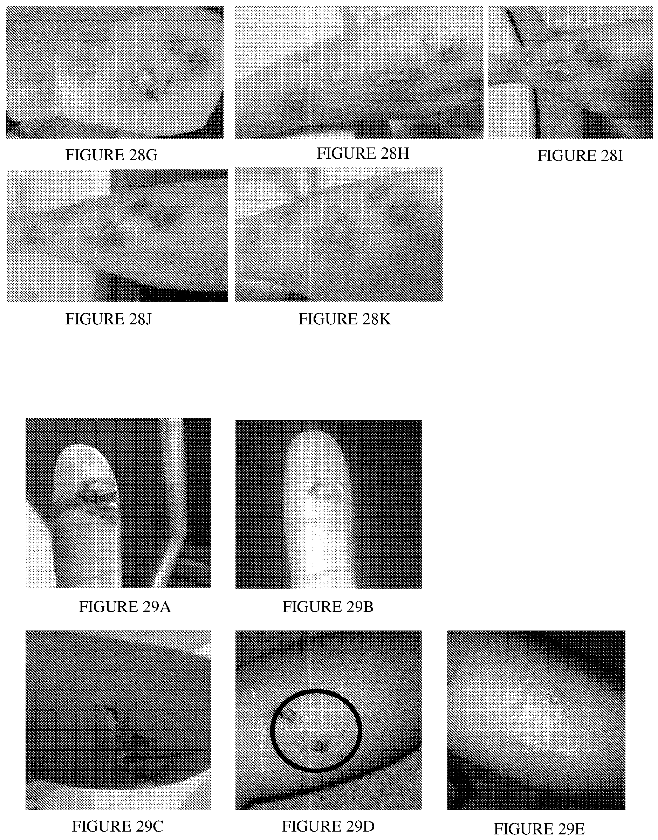

**Figs. 28A-28K** are image depicting the healing of Leishmaniosis ulcers after application of the herein disclosed probiotic yogurt. After conventional treatment has failed to improve healing of the ulcers (included PENTOSTAM injection after local anesthesia with ESRACAIN, and topical administration of SALIKAREN), the probiotic yogurt was applied topically (twice a day, e.g., in the morning and in the evening). (**28A**, **28G**) day 0, (**28B, 28H**) day 1, (**28C**, **281**) day 2, (**28D**) day 4, (**28J**) day 5, (**28E**) day 6, (**28F**) day 11, and (**28K**) day 14, after application of the probiotic yogurt commenced. Accelerated healing of cutaneous leishmaniosis were evident on day 11 and day 14 onwards (**Figs. 28F**, and **28K**, respectively).
**Figs. 29A-29E** are images showing wounds healing after application of the herein disclosed probiotic yogurt. Infected wounds were evident on day 0 (**29A**, and **29C**). In (**29A**) sutures were required for the fusion of the incision, but as they were not performed because more than 24 hours have passed since the injury, the herein disclosed probiotic yogurt was topically applied, and the wound healed after four days (**29B**). Growth of new tissue was evident two days after application of the probiotic yogurt had commenced (**29D**), and onwards. (**29E**) day 0, (**29B**) day 4, (**29D**) day 2, and (**29E**) day 7, after application of the probiotic yogurt commenced.

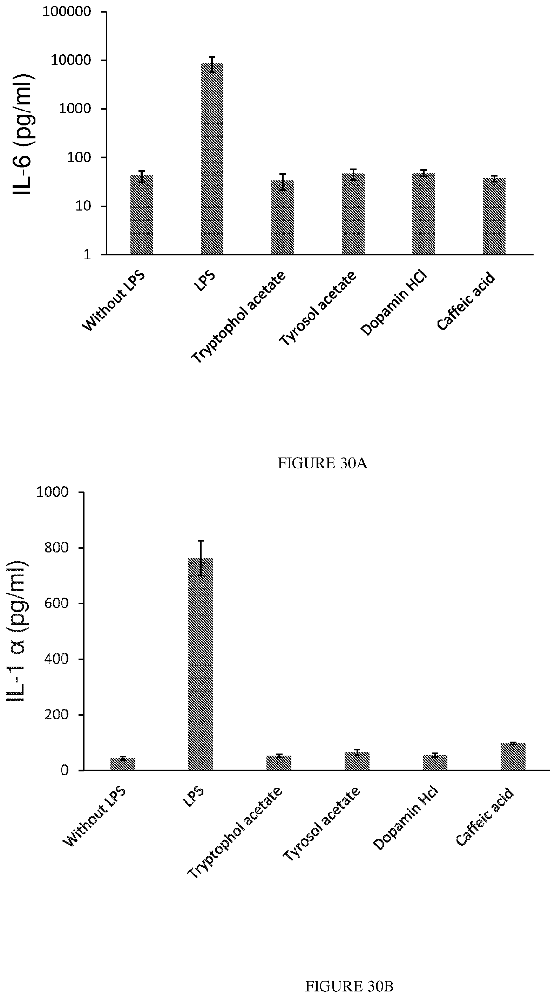

**Figs. 30A-30B** are vertical bar graphs showing an anti-inflammatory effect induced by molecules identified in the herein disclosed probiotic yogurt. Production of IL-6 (**30A**) and IL-1α (**30B**) cytokines by macrophages were dramatically reduced in presence of Tryptophol acetate and 4-Ethyl-Phenol derivatives (e.g., Tyrosol acetate, dopamine HCl, and caffeic acid). Lipopolysaccharides (LPS).
**Fig**. **31** is a graph showing the results of thioflavin T (ThT) assay examining the effect of a mixture of molecules extracted from the herein disclosed probiotic yogurt (e.g., kefir), and tryptophol acetate on amyloid beta (αβ 1-42) fibrillation. Results show that both the molecules extracted from the herein disclosed probiotic yogurt (e.g., kefir), and tryptophol acetate in varying concentrations reduced αβ 1-42 fibrillation. Mix - a mixture of molecules extracted from the yogurt; OM - tryptophol acetate.

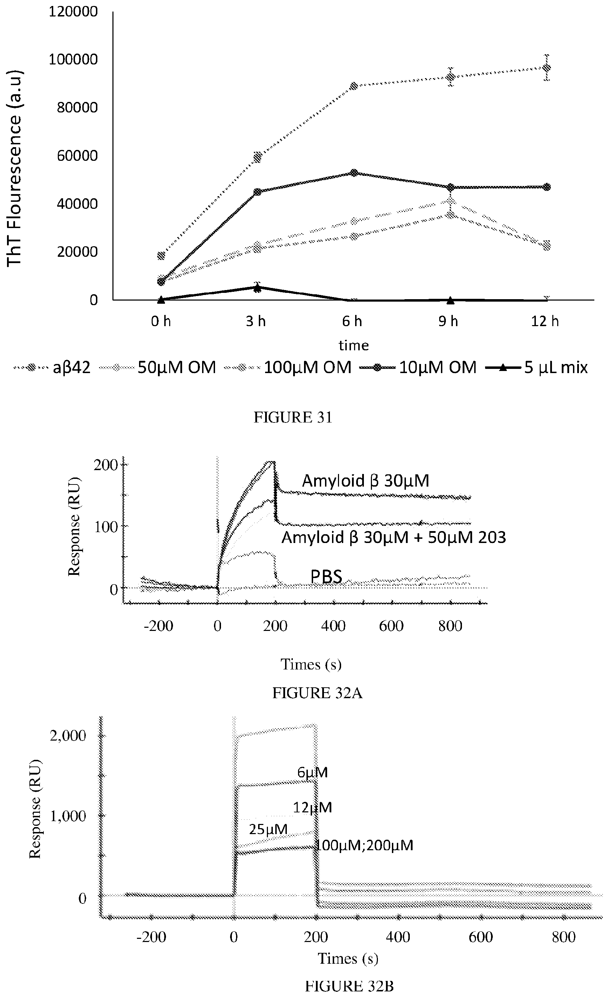

**Figs. 32A-32B** are graphs showing αβ 1-42 fibrillation in the absence or presence of tryptophol acetate. (**32A**) a graph of surface plasmon resonance (SPR) showing fibrillation degree of 30 µM αβ 1-42 alone (L1A1-2 - AmyloidB), 30 µM αβ 1-42 suspended in PBS (L1A3-4 - bf); or 30 µM αβ 1-42 in the presence of 50 µM tryptophol acetate (L1A5-6 - AmyloidB + 203). (**32B**) a graph of SPR showing fibrillation degree of 30 µM αβ 1-42 in the presence of several tryptophol acetate concentrations (6 µM, 12 µM, 25 µM, 100 µM, and 200 µM).

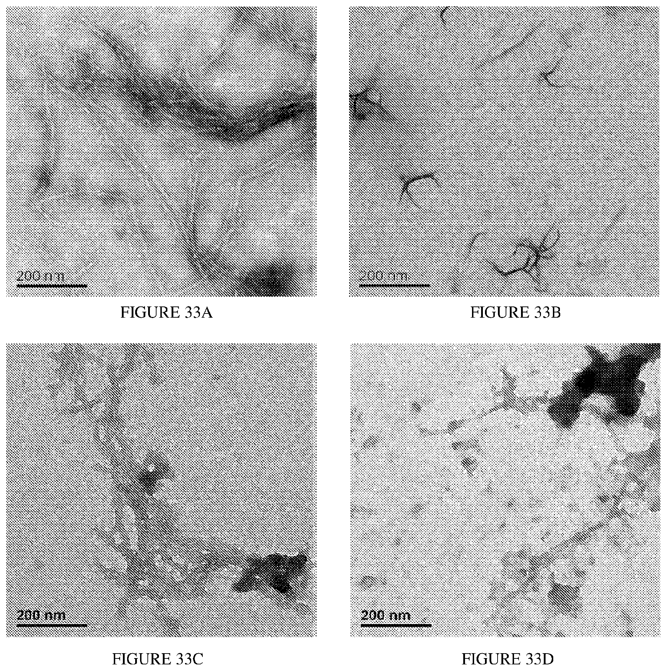

**Figs. 33A-33D** are micrographs of transmission electron microscopy (TEM). αβ 1-42 was incubated in 37 °C for 24 hr either alone (**33A**), or in presence of 5µl of the herein disclosed probiotic yogurt crude extract (**33B**), 25 µM of tryptophol acetate (**33C**), or 50 µM of tryptophol acetate (**33D**).

### DETAILED DESCRIPTION OF THE INVENTION

In some embodiments, the present invention is directed to antimicrobial molecules selected from derivatives of Tryptophol and/or 4-Ethyl-Phenol, compositions and devices comprising same, and methods of use thereof including but not limited to inhibition of biofilm formation, as defined in the claims.

In some embodiments, the present invention is directed to a method for treating a disease selected from the group consisting of: an inflammatory disease, an infectious disease, and an amyloid aggregates-related disease, in a subject in need thereof, by administering to the subject a therapeutically effective amount of a pharmaceutical composition comprising any one of: at least one of a Tryptophol derivative, a 4-Ethyl-Phenol derivative, and a microorganism mixture comprising: K. marxianus; and at least one probiotic microorganism, wherein at least 3% of cells in the microorganism mixture are K. marxianus.

In some embodiments, the present invention is directed to a method for inhibiting or reducing amyloid (e.g., amyloid beta) aggregates, the method comprising contacting a surface or a cell with an effective amount of any one of: Tryptophol derivative, 4-Ethyl-Phenol derivative, and the microorganism mixture described herein. In some embodiments, amyloid aggregation comprises amyloid fibrillation, amyloid oligomerization, or a combination thereof.

In some embodiments, reducing the load of an organic-based contaminant comprises inhibiting or reducing amyloid.

As used herein, the terms "amyloid aggregates" and "amyloid aggregation" refers to aggregates or clumps of an amyloid protein which refold into conformations allowing multiple copies to aggregate, thereby yielding aggregations, fibrils, oligomers, or any combination thereof. Types of protein featured in amyloids would be apparent to one of ordinary skill in the art. In some embodiments, an amyloid comprises beta amyloid.

In some embodiments, any one of the molecules of the invention, or the microorganism mixture (e.g., kefir) of the invention, have any one of: antimicrobial activity, anti-inflammatory activity, and anti-amyloid aggregation activity.

As used herein, the term "anti-amyloid aggregation activity" refers to the ability to prevent or reduce amyloid dimerization, amyloid oligomerization, amyloid aggregation, amyloid sedimentation, amyloid unfolding, amyloid non-native folding, or any combination thereof.

As used herein, the term "antimicrobial activity" refers to the ability to inhibit, prevent, reduce or retard bacterial growth, fungal growth, biofilm formation, amyloid aggregation, e.g., amyloid beta, or eradicate living bacterial cells, or their spores, or fungal cells or viruses in a suspension, on a surface or in a moist environment. In some embodiments, inhibiting or reducing or retarding the formation of load of a microorganism refers to inhibiting reducing or retarding growth of microorganisms, biofilm production by microorganisms, production or aggregation of amyloids, e.g., amyloid beta, and/or eradicating a portion or all of an existing population of microorganisms.

As used herein, the term "anti-inflammatory" refers to the ability to inhibit, prevent, reduce or retard inflammatory response of the body, including cytokine production, secretion, or both, immune cell priming, homing, activation, recruitment, or any combination thereof. In some embodiments, an inflammatory response comprises an autoinflammatory response.

The invention is based, in part, on the finding the derivative of Tryptophol, Tryptophol acetate, and the derivative of 4-Ethyl-Phenol, Tyrosol acetate, modified microorganisms' quorum sensing (both bacteria and fungi), and reduced biofilm production and fungal growth. The anti-biofilm effect, as well as the anti-inflammatory, and anti-amyloid aggregation effect was demonstrated using Tryptophol and/or Tyrosol derivatives obtained from the microorganism culture described herein as well as synthetic Tryptophol and/or Tyrosol derivatives.

As used herein, the term "compound or molecule of the invention" refers to Tryptophol acetate and Tyrosol acetate, having antimicrobial activity, anti-inflammatory activity, and anti-amyloid aggregation activity. The invention, however, concerns a composition comprising both compounds, as further defined in the claims.

### Tryptophol derivative

The Tryptophol derivative according to the invention is Tryptophol acetate.

Tryptophol acetate is known in the art as having the structure:

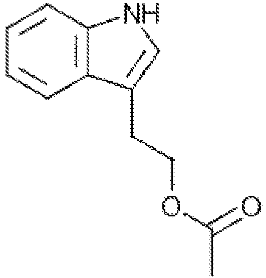

### 4-Ethyl-Phenol derivative

The 4-Ethyl-Phenol derivative of the invention is Tyrosol acetate.

Tyrosol acetate is known in the art as having the structure:

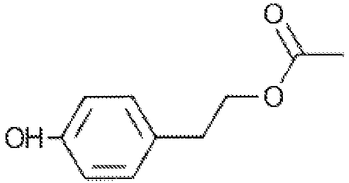

As used herein, the term "derivative" encompasses any compound having antimicrobial activity that is generated from a similar compound by a chemical reaction, or any compound produced from another compound by substitution of one or more atoms. In some embodiments, the derivative comprises a structural analog.

As used herein, the compound of the invention does not comprise Tryptophol or Tyrosol.

The disclosed invention is directed to a composition comprising Tryptophol acetate and Tyrosol acetate, and at least one pharmaceutically acceptable carrier or diluent.

In some embodiments, the compound of the invention is chemically synthesized or biosynthesized. Methods of biosynthesis are well known within the art, and can include, but are not limited to: production in a cell culture or enzymatic cell-free production. In some embodiments, the compound of the invention is biosynthesized using a cell culture comprising Kluyveromyces marxianus. In some embodiments, the culture comprising K. marxianus is a mono- or poly-culture. In some embodiments, the compound of the invention is biosynthesized by K. marxianus. In some embodiments, Tryptophol acetate or Tyrosol acetate are biosynthesized by K. marxianus according to the method of the present invention.

### Microorganism mixture

In some embodiments, the present invention is directed to a composition comprising a mixture of microorganisms. In some embodiments, the composition comprises probiotic microorganisms. In some embodiments, a probiotic microorganism comprises yeasts. In some embodiments, the majority of microorganisms within the mixture of the disclosed invention is of probiotic yeast. In some embodiments, the probiotic yeast of the disclosed invention is Kluyveromyces marxianus.

In one embodiment, K. marxianus is K. marxianus strain HA 63 [NRRL Y-8281, CBS 712].

As used herein, the term "probiotic" refers to any substance and/or a microorganism that promotes growth, especially of microorganisms with beneficial properties (e.g., intestinal flora).

In some embodiments, a microorganism content (%) within the mixture of the present invention is quantified according to the portion of the microorganism's DNA out of the total DNA of the mixture. In some embodiments, DNA quantification is based directly on the amount of DNA extracted. In some embodiments, DNA quantification further comprises enzymatic reaction, including but not limited to restriction, ligation, amplification, sequencing, or any combination thereof. In some embodiments, DNA quantification is based on next generation sequencing. In some embodiments, DNA quantification is based on the ratio of the amount of microorganism-specific DNA reads compared to the total number of DNA reads of the microorganism mixture.

In some embodiments, a microorganism content within the mixture of the present invention comprises the number of cells of the microorganism per volume of the mixture culture (e.g., colony forming unit [CFU]). In some embodiments, a microorganism content within the mixture of the present invention comprises the number of cells of the microorganism compared to the total number of cells in the mixture. In some embodiments, a microorganism content within the mixture of the invention comprises the CFU of the microorganism.

In some embodiments, at least 1%, at least 3%, at least 5%, at least 7%, at least 10%, at least 15%, at least 20%, at least 30%, at least 35%, at least 40%, at least 45%, at least 50%, at least 55%, at least 60%, at least 65%, or at least 70% of cells in the microorganism mixture are K. marxianus cells. In some embodiments, 1-4%, 2-5%, 4-7%, 6-11%, 10-16%, 15-22%, 20-32%, 30-35%, 32-40%, 38-48%, 45-55%, 50-60%, or 55-75%, 60-80%, 65-90%, or 80-100% of cells in the microorganism mixture are K. marxianus cells. Each possibility represents a separate embodiment of the invention.

In some embodiments, the composition of the disclosed invention comprises at least one probiotic bacterium. In one embodiment, a probiotic bacterium is selected from the genus Lactobacillus. In one embodiment, a probiotic bacterium is selected from the genus Propionibacterium. In one embodiment, a probiotic bacterium is selected from the genus. In one embodiment, a probiotic bacterium is selected from the genus Lactococcus. In one embodiment, a probiotic bacterium is selected from the genus Leuconostoc. In some embodiments, at least one probiotic bacterium is at least two, at least three, at least four, at least five, at least six, at least seven, at least eight, at least nine or at least ten probiotic bacteria. In some embodiments, 30% at most, 25% at most, 20% at most, 15% at most, 10% at most, 5% at most, or 1% at most, of cells in the microorganism mixture are probiotic bacteria. In some embodiments, 1-5%, 4-10%, 8-18%, 12-20%, 17-25%, or 22-30% of cells in the microorganism mixture are probiotic bacteria. Each possibility represents a separate embodiment of the invention.

In some embodiments, the microorganism mixture of the disclosed invention further comprises other types of microorganisms. In one embodiment, the other microorganisms are not probiotic microorganisms, such as yeast or bacteria. In some embodiments, 15% at most, 13% at most, 11% at most, 10% at most, 9% at most, 7% at most, 5% at most, 4% at most, 3% at most, 2% at most, or 1% at most of cells in the microorganism mixture belong to a microorganism type other than a probiotic yeast and at least one probiotic bacteria. In some embodiments, the microorganism mixture comprises no other type of microorganism except probiotic yeast and at least one probiotic bacteria.

In some embodiments, the microorganism mixture is suspended in a medium. In some embodiments, the microorganism mixture is grown in the medium. In some embodiments, the medium is a cell culture medium suitable for growth and maintenance of the microorganism mixture. In one embodiment, the cell culture medium is optimized for microorganism growth, such as milk.

As used herein, "cell culture medium" refers to any medium, liquid or solid, which enables cells proliferation. Cell culture media are known in the art and can be selected depending of the type of cell to be grown. For example, a cell culture medium for use in growing cells is Luria-Bertani broth (LB; Miller's broth). In some embodiments, microorganism mixture is cultured under effective conditions, which allow for increased yield of production from the culture microorganism mixture. Non-limiting example for increased yield include, but not limited to, increased gene expression, protein production and secretion, molecule biosynthesis, proliferation and others. In some embodiments, effective culture conditions include, but are not limited to, effective media, bioreactor, temperature, pH and oxygen conditions that permit for increased production yield. In one embodiment, an effective medium refers to any medium in which a microorganism mixture is cultured to produce a compound of the present invention. In some embodiments, a cell culture medium typically includes an aqueous solution having assimilable carbon, nitrogen and phosphate sources, and appropriate salts, minerals, metals and other nutrients, such as vitamins. In some embodiments, microorganism mixture of the present invention can be cultured in conventional fermentation bioreactors, shake flasks, test tubes, microtiter dishes and petri plates. In some embodiments, culturing is carried out at a temperature, pH and oxygen content appropriate for a probiotic microorganism, such as a yeast or bacteria. In some embodiments, culturing conditions are within the expertise of one of ordinary skill in the art. A non-limiting example for a process of culturing a microorganism mixture of the present invention comprises culturing the microorganism mixture in milk at 28 °C for about 24 hours.

In one embodiment, the process of culturing comprises culturing the microorganism mixture for a period of 12-16 hours, 14-18 hours, 12-24 hours, 16-24 hours, 18-28 hours, 10-20 hours, 22-36 hours. Each possibility represents a separate embodiment of the invention.

In one embodiment, the process of culturing comprises culturing the microorganism mixture at a temperature of 20-26 °C, 24-28 °C, 22-34 °C, 26-34 °C, 28-38 °C, 20-30 °C, 32-46 °C. Each possibility represents a separate embodiment of the invention.

In some embodiments, a microorganism mixture of the invention is cultured in milk. In some embodiments, a microorganism mixture cultured in milk yields a fermented milk product. In some embodiments, the fermented milk product is selected from: yogurt, probiotic yogurt or kefir.

In some embodiments, the fermented milk product of the present invention comprises a microorganism mixture, Tryptophol derivative, or Tyrosol derivative or any combination thereof.

In some embodiments, the fermented milk product of the present invention is for human foodstuff consumption. In some embodiments, the fermented milk product of the present invention is consumed by a subject as part of a daily consumption. In one embodiment, the fermented milk product is consumed by a subject as part of a dietary consumption. In some embodiments, the fermented milk product can be used to treat skin lesions. In some embodiments, the fermented milk product is applied topically to a subject.

In some embodiments, the present invention provides a method for producing or obtaining the herein disclosed microorganism mixture. In some embodiments, the method comprises culturing K. marxianus as described herein. In some embodiments, the method further comprises a step of determining that the herein disclosed microorganism mixture was obtained or produced. In some embodiments, determining comprises determining that at least 3% of the cells in the mixture are K. marxianus. In some embodiments, determining comprises determining that a tryptophol derivative or a 4-Ethyl-Phenol derivative is produced. In some embodiments, determining comprises determining that a tryptophol derivative and a 4-Ethyl-Phenol derivative are produced. In some embodiments, determining comprises determining that at least 3% of the cells in the mixture are K. marxianus and a tryptophol derivative or a 4-Ethyl-Phenol derivative are produced in sufficient amounts. In some embodiments, determining comprises determining that at least 3% of the cells in the mixture are K. marxianus and a tryptophol derivative and a 4-Ethyl-Phenol derivative are produced in sufficient amounts. As used herein, sufficient amounts comprises therapeutically effective amounts as described herein, such as having or eliciting anti-inflammatory response, antimicrobial activity, anti-amyloid aggregation activity, or a combination thereof.

### Methods of use and compositions

According to some embodiments, there is provided a method for a disease selected from the group consisting of: an inflammatory disease, an infectious disease, and an amyloid aggregates-related disease, in a subject in need thereof, the method comprising administering to said subject a therapeutically effective amount of a pharmaceutical composition comprising any one of: at least one molecule selected from a Tryptophol derivative and a 4-Ethyl-Phenol derivative, as defined in the claims, and a microorganism mixture as disclosed herein.

In some embodiments, the infectious disease comprises an organic-based contaminant. In some embodiments, the organic-based contaminant comprises a biofilm.

In some embodiments, an organic-based contaminant comprises amyloid aggregation, e.g., amyloid beta.

As used herein, the term "organic" refers to any one of: a compound comprising carbon, a matter produced or derived from an organism, an organ, and an organism.

In some embodiment, contacting is administering. In some embodiments, contacting is incorporating. In some embodiment, contacting is administering to a subject. In some embodiments, contacting is incorporating to a surface. In some embodiments, contacting is contacting a cell. In some embodiments, a cell is a unicellular microorganism. In some embodiments, a cell is a cell of a subject. In some embodiments, a cell is a cell of the nerve system. In some embodiments, a cell of the nerve system is a neuron cell.

In some embodiments, the organic-based contaminant is on or within a surface, an article, a cell, or a subject. In some embodiments, the subject comprises the cell. In some embodiments, the cell is an endogenous cell or an exogenous cell.

In some embodiments, there is provided a method for treating a biofilm-related infectious disease or a symptom thereof in a subject in need thereof, the method comprising administering to a subject a therapeutically effective amount of pharmaceutical composition comprising at least one molecule selected from Tryptophol derivative or a 4-Ethyl Phenol derivative, or a microorganism mixture as disclosed herein, and at least one pharmaceutically acceptable carrier.

As used herein, "organic-based contaminant-related infectious disease" refers to any disease or disorder caused to a subject by an increased load of a microorganism, and/or a formation of a biofilm or biofouling. In some embodiments, an organic-based contaminant-related infectious disease inducing organism is selected from the group consisting of: bacteria, viruses, fungi or parasites.

Non-limiting examples for symptoms of an infectious disease include, but are not limited to, fever, diarrhea, fatigue, muscle aches and coughing.

Non-limiting examples of infectious diseases include urinary tract infection, gastrointestinal infection, enteritis, salmonellosis, diarrhea, nontuberculous mycobacterial infections, legionnaires' disease, hospital-acquired pneumonia, skin infection, cholera, septic shock, periodontitis, infection, inflammatory bowel disease, ulcerative colitis (UC), Crohn's disease, and sinusitis. In some embodiments, the infection induces a condition selected from the group consisting of: bacteremia, skin infections, neonatal infections, pneumonia, endocarditis, osteomyelitis, toxic shock syndrome, scalded skin syndrome, and food poisoning.

The term "subject" as used herein refers to an animal, more particularly to non-human mammals and human organism. Non-human animal subjects may also include prenatal forms of animals, such as, e.g., embryos or fetuses. Non-limiting examples of non-human animals include: horse, cow, camel, goat, sheep, dog, cat, non-human primate, mouse, rat, rabbit, hamster, guinea pig, and pig. In one embodiment, the subject is a human. Human subjects may also include fetuses.

As used herein, the terms "treatment" or "treating" of a disease, disorder, or condition encompasses alleviation of at least one symptom thereof, a reduction in the severity thereof, or inhibition of the progression thereof. Treatment need not mean that the disease, disorder, or condition is totally cured. To be an effective treatment, a useful composition herein needs only to reduce the severity of a disease, disorder, or condition, reduce the severity of symptoms associated therewith, or provide improvement to a patient or subject's quality of life.

As used herein, the term "prevention" of a disease, disorder, or condition encompasses the delay, prevention, suppression, or inhibition of the onset of a disease, disorder, or condition. As used in accordance with the presently described subject matter, the term "prevention" relates to a process of prophylaxis in which a subject is exposed to the presently described peptides prior to the induction or onset of the disease/disorder process. This could be done where an individual has a genetic pedigree indicating a predisposition toward occurrence of the disease/disorder to be prevented. For example, this might be true of an individual whose ancestors show a predisposition toward certain types of, for example, inflammatory disorders. The term "suppression" is used to describe a condition wherein the disease/disorder process has already begun but obvious symptoms of the condition have yet to be realized. Thus, the cells of an individual may have the disease/disorder, but no outside signs of the disease/disorder have yet been clinically recognized. In either case, the term prophylaxis can be applied to encompass both prevention and suppression. Conversely, the term "treatment" refers to the clinical application of active agents to combat an already existing condition whose clinical presentation has already been realized in a patient.

As used herein, the term "condition" includes anatomic and physiological deviations from the normal that constitute an impairment of the normal state of the living animal or one of its parts, that interrupts or modifies the performance of the bodily functions.

In some embodiments, a composition as disclosed herein is directed to killing microorganisms in a living tissue or on or in an article or reducing the formation of microorganisms on or in an article.

In some embodiments, the present invention is directed to a method of inhibiting or reducing a formation of load of organic-based contaminant on or within an article, comprising incorporating or coating the composition comprising at least one molecule selected from Tryptophol derivative and 4-Ethyl Phenol derivative, or a microorganism mixture as disclosed herein, and an acceptable carrier, on and/or within an article.

In some embodiments, the load of organic-based contaminant is a load of a microorganism, and/or a formation of a biofilm or biofouling in and/or on an article. In some embodiments, the microorganism is selected form the group consisting of: a virus, a fungus, a parasite, a yeast, a bacterium, and a protozoa.

According to some embodiments, the method comprises treating or ameliorating an organic-based contaminant-related infectious disease or a symptom thereof in a subject in need thereof, comprising administering to the subject any one of: the compound of the invention or a pharmaceutical composition comprising thereof; or the microorganism mixture as disclosed herein, or a fermented milk product comprising thereof.

In some embodiments, there is provided a use of a composition comprising an effective amount of the compound of the invention, or a microorganism mixture of the invention, in the preparation of a medicament for the treatment, amelioration, reduction, or prevention of an organic-based contaminant-related infectious disease or a symptom thereof in a subject in need thereof. In some embodiments, the invention provides use of a composition comprising an effective amount of one or more of the molecules or any derivative thereof, or a microorganism mixture as disclosed herein, in the preparation of a medicament for the treatment of an infectious disease or a symptom thereof in a subject in need thereof.

In one embodiment, the compound of the invention, or the microorganism mixture of the invention, is provided to the subject per se. In one embodiment, one or more of the compounds of the invention are provided to the subject per se. In one embodiment, the compound of the invention, or the microorganism mixture of the invention is provided to the subject as part of a pharmaceutical composition where it is mixed with a pharmaceutically acceptable carrier. In one embodiment, one or more of the compounds of the invention are provided to the subject as part of a pharmaceutical composition where they are mixed with a pharmaceutically acceptable carrier.

As used herein" the term "quorum sensing (QS)" refers to systems which modify gene expression pathways in response to dynamics of cell-population density. In some embodiments, molecules of the present invention or any derivative thereof , are used in a method for inhibiting QS. Any method known in the art can be used for evaluating the effect of a molecule on QS. A non-limiting example for QS examination comprises the use of a bioluminescence assay based on engineered bacteria lacking an active autoinducer gene and further cloned with a DNA vector comprising a fluorescent reporter gene.

As used herein, the term "biofilm" refers to a group of microorganisms adhering to one another, which are embedded within self-produced and self-secreted extracellular polymer comprising DNA, proteins and polysaccharides. In some embodiments, a biofilm adheres to a surface on a living host. In one embodiment, a biofilm adheres to a non-living surface. In some embodiments, QS activity correlates with level of biofilm formation.

In some embodiments, any one of the compositions, fermented milk product or method of the present invention modifies QS activity by at least 1%, at least 5%, at least 10%, at least 20%, at least 30, at least 40%, at least 50%, at least 60%, at least 70%, at least 80%, at least 90%, or at least 100%, compared to control, or any value and range therebetween. Each possibility represents a separate embodiment of the invention. In some embodiment, modifying QS activity is by 1-5%, 3-6%, 4-10%, 8-20%, 15-30, 28-40%, 35-50%, 45-60%, 50-70%, 65-80%, 70-90%, or 90-100%, compared to control. In some embodiment, modifying QS activity is by at least 2-fold, at least 3-fold, at least 4-fold, at least 5-fold, at least 6-fold, at least 7-fold, at least 8-fold, at least 9-fold, or at least 10-fold, compared to control. Each possibility represents a separate embodiment of the invention.

In some embodiments, any one of the: compositions, fermented milk product or method of the present invention reduce biofilm production by at least 1%, at least 5%, at least 10%, at least 20%, at least 30, at least 40%, at least 50%, at least 60%, at least 70%, at least 80%, at least 90%, or at least 100%, compared to control, or any value and range therebetween. Each possibility represents a separate embodiment of the invention. In some embodiment, biofilm production is reduced by 1-5%, 3-6%, 4-10%, 8-20%, 15-30%, 28-40%, 35-50%, 45-60%, 50-70%, 65-80%, 70-90%, or 90-100%, compared to control. In some embodiment, biofilm production is reduced by at least 2-fold, at least 3-fold, at least 4-fold, at least 5-fold, at least 6-fold, at least 7-fold, at least 8-fold, at least 9-fold, or at least 10-fold, compared to control, or any value and range therebetween. Each possibility represents a separate embodiment of the invention.

In one embodiment, a control is a non-fermented milk product. In one embodiment, a control is a fermented milk product having a different microorganism mixture compared to the microorganism mixture of the disclosed invention. In one embodiment, a control is a fermented or non-fermented milk product devoid of K. marxianus. In one embodiment, a control is a fermented or non-fermented milk product comprising a microorganism mixture comprising 2% or less K. marxianus. In one embodiment, a control is a fermented or non-fermented milk product comprising a microorganism mixture comprising inactivated K. marxianus. Non-limiting example of inactivation is heat-inactivation. In some embodiments, a control comprises Tyrosol, or Tryptophol, or any combination thereof, as disclosed above. In some embodiments, a control does not comprise Tyrosol derivative or Tryptophol derivative, or a combination thereof.

In some embodiments, the invention is directed to a composition as defined in the claims comprising as an active ingredient an effective amount of the compound of the invention, and an acceptable carrier and/or diluent. In some embodiments, the invention is directed to a composition comprising as an active ingredient an effective amount of Tryptophol acetate, Tyrosol acetate, dopamine HCL, caffeic acid, or any combination thereof. In some embodiments, the acceptable carrier facilitates incorporation or coating of the active ingredient a substrate.

In some embodiments, a composition, further comprises a substrate. In some embodiments a composition comprising at least one of: Tryptophol derivative or 4-Ethyl Phenol derivative, is incorporated in and/or on at least a portion of the substrate.

In some embodiments, the invention is directed to a composition comprising a substrate having incorporated in and/or on at least a portion thereof, at least one of: Tryptophol derivative or 4-Ethyl Phenol derivative.

As used herein, the term "a portion thereof" refers to, for example, a surface or a portion thereof, and/or a body or a portion thereof, of solid or semi-solid substrates; or a volume or a part thereof, of liquid, gel, foams and other non-solid substrates.

Substrates of widely different chemical nature can be successfully utilized for incorporating (e.g., depositing on a surface thereof) at least one of: Tryptophol derivative or 4-Ethyl Phenol, or a composition comprising thereof, thereon, as described herein. The term "successfully utilized" refers to an outcome meaning that: (i) at least one of: Tryptophol derivative or Tyrosol derivative, or a composition comprising thereof, successfully formed a uniform and homogenously coating on the substrate's surface; and (ii) the resulting coating imparts long-lasting desired properties (e.g., antimicrobial properties) to the substrate's surface.

Substrate usable according to some embodiments of the present invention can therefore be hard (rigid) or soft, solid, semi-solid, or liquid substrates, and may take a form of a foam, a solution, an emulsion, a lotion, a gel, a cream or any mixture thereof.

Substrate usable according to some embodiments of the present invention can have, for example, organic or inorganic surfaces, including, but not limited to, glass surfaces; porcelain surfaces; ceramic surfaces; silicon or organosilicon surfaces, metallic surfaces (e.g., stainless steel); mica, polymeric surfaces such as, for example, plastic surfaces, rubbery surfaces, paper, wood, polymer, a metal, carbon, a biopolymer, silicon mineral (rock or glass), surfaces, wool, silk, cotton, hemp, leather, fur, feather, skin (hide, pelt or pelage) surfaces, plastic surfaces and surfaces comprising or made of polymers such as but not limited to polypropylene (PP), polycarbonate (PC), polyethylene (PET), high-density polyethylene (HDPE), low-density polyethylene (LDPE), polyester (PE), unplasticized polyvinyl chloride (PVC), and fluoropolymers including but not limited to polytetrafluoroethylene (PTFE, Teflon^{®}); or can comprise or be made of any of the foregoing substances, or any mixture thereof.

Alternatively, other portions, or the entire substrate are made of the above-mentioned materials.

In some embodiments, the substrate incorporating at least one of: Tryptophol derivative or 4-Ethyl Phenol derivative, or a composition comprising thereof, as described herein is or forms a part of an article.

According to some embodiments, an article (e.g., an article-of-manufacturing) comprises a substrate incorporating in and/or on at least a portion thereof at least one of: Tryptophol derivative or 4-Ethyl Phenol derivative or a composition comprising thereof.

The article can be any article which can benefit from the antimicrobial and/or anti-biofilm formation activities of Tryptophol derivative or 4-Ethyl Phenol derivative.

Non-limiting examples of articles include, but are not limited to, medical devices, organic waste processing device, fluidic device, an agricultural device, a package, a sealing article, a fuel container, a water and cooling system device and a construction element.

Non-limiting examples of devices which can incorporate at least one of: Tryptophol derivative or 4-Ethyl Phenol derivative or a composition comprising thereof, as described herein, beneficially, include tubing, pumps, drain or waste pipes, screw plates, and the like.

Non-limiting example of an article include but is not limited to an element used in water treatment systems (such as for containing and/or transporting and/or treating aqueous media or water), devices, containers, filters, tubes, solutions and gases and the likes.

Non-limiting example of an article include but is not limited to an element in organic waste treatment systems (such as for containing and/or disposing and/or transporting and/or treating organic waste), devices, containers, filters, tubes, solutions and gases and the likes.

In some embodiments, the invention is directed to a pharmaceutical composition comprising as an active ingredient a therapeutically effective amount of the compound of the invention or the microorganism mixture of the invention, and a pharmaceutically acceptable carrier and/or diluent. Specifically, , the invention is directed to a pharmaceutical composition comprising as active ingredients a therapeutically effective amount of Tryptophol acetate and Tyrosol acetate. In some embodiments, the pharmaceutically acceptable carrier facilitates administration of the compound of the invention to an organism. For example, the term "pharmaceutically acceptable" can mean approved by a regulatory agency of the Federal or a state government or listed in the U.S. Pharmacopeia or other generally recognized pharmacopeia for use in animals, and more particularly in humans.

In some embodiments, the disclosed invention is directed to a composition for use in modifying pathogenic activity, microbial growth, microbial activity, modifying inflammatory response, modifying amyloid aggregation, or any combination thereof. As defined herein, "modifying" encompasses increasing or decreasing, depending on the desired outcome. In some embodiments, pathogenic activity comprises microbial activity. In some embodiments, microbial activity comprises any activity selected from: proliferation, antibiotic resistance, cell communication or quorum sensing, biofilm production, amyloid aggregation, fibrillation or oligomerization, or any combination thereof, toxin production or secretion, or combination thereof. Microbial activity can be assayed using any known method, non-limiting examples include, but are not limited to, spectrophotometry, drug resistance assays using selective substrates, bioluminescence assay, liquid chromatography and mass-spectrometry or others, some of which are exemplified herein below, and all of which are well known to one of ordinary skill in art.

In some embodiments, the disclosed invention is directed to a method for treating an inflammatory disease, an infectious disease, an amyloid aggregation-related disease, or a combination thereof.

As used herein, the term "amyloid aggregation-related disease" encompasses any pathogenic condition comprising amyloid involvement as part of the disease pathogenesis or pathophysiology. In some embodiments, amyloid-related disease comprises any one of: elevated amyloid aggregation, elevated amyloid production rates, elevated amyloid fibrillation rates, elevated amyloid oligomerization rates, amyloid-induced toxicity, amyloid-induced apoptosis, amyloid-induced cell death, and any combination thereof.

In some embodiments, the amyloid aggregation-related disease is a neurodegenerative disease. As used herein, neurodegenerative disease may also include neuro-muscular degenerative diseases. None limiting examples include autonomic neuropathies, Horner syndrome, multiple system atrophy, pure autonomic failure, delirium, dementia, Alzheimer's disease, chronic traumatic encephalopathy, frontotemporal dementia, Lewy body dementia, Parkinson disease, multiple sclerosis, neuromyelitis optica, Huntington's disease, progressive supranuclear palsy, neuro-ophthalomologic and cranial nerve disorder, Isaacs Syndrome, Stiff-Person syndrome, Guillain-Barré syndrome (GBS), chronic inflammatory demyelinating polyneuropathy (CIDP), hereditary neuropathies, hereditary motor neuropathy with liability to pressure palsies (HNPP), amyotrophic lateral sclerosis (ALS) and other motor neuron diseases (MNDs), myasthenia gravis, nerve root disorders, herniated nucleus pulposus, peripheral neuropathy, mononeuropathies, multiple mononeuropathy, polyneuropathy, brachial plexus and lumbosacral plexus disorders, spinal muscular atrophies (SMAs), thoracic outlet compression syndromes (TOS), Creutzfeldt-Jakob Disease (CJD), Gerstmann-Sträussler-Scheinker Disease (GSS), seizure disorders, spinal cord disorders and stroke.

In some embodiments, modifying is by at least 5%, at least 15%, at least 25%, at least 40%, at least 50%, at least 65%, at least 75%, at least 85%, at least 90%, at least 95%, or at least 99%, or any value and range therebetween. Each possibility represents a separate embodiment of the invention. In some embodiments, modifying is by 1-5%, 7-15%, 10-25%, 20-40%, 35-50%, 45-65%, 55-75%, 70-85%, 80-90%, 87-95%, or 92-100%. Each possibility represents a separate embodiment of the invention.

In some embodiments, reducing is by at least 5%, at least 15%, at least 25%, at least 40%, at least 50%, at least 65%, at least 75%, at least 85%, at least 90%, at least 95%, or at least 99%, or any value and range therebetween. Each possibility represents a separate embodiment of the invention. In some embodiments, reducing is by 1-5%, 7-15%, 10-25%, 20-40%, 35-50%, 45-65%, 55-75%, 70-85%, 80-90%, 87-95%, or 92-100%. Each possibility represents a separate embodiment of the invention.

The terms "reduce" and "inhibit" are used herein interchangeably.

In some embodiments, enhancing is by at least 5%, at least 15%, at least 25%, at least 40%, at least 50%, at least 65%, at least 75%, at least 85%, at least 90%, at least 95%, or at least 99%, or any value and range therebetween. Each possibility represents a separate embodiment of the invention. In some embodiments, enhancing is by 1-5%, 7-15%, 10-25%, 20-40%, 35-50%, 45-65%, 55-75%, 70-85%, 80-90%, 87-95%, or 92-100%. Each possibility represents a separate embodiment of the invention.

The composition described herein comprises the Tryptophol derivative Tryptophol acetate and the 4-Ethyl Phenol derivative Tyrosol acetate in 1:10 to 10:1 w/w ratio. In some embodiments, the composition described herein comprises Tryptophol derivative or 4-Ethyl Phenol derivative in 1:9 to 9:1 w/w ratio. In some embodiments, the composition described herein comprises Tryptophol derivative or 4-Ethyl Phenol derivative in 1:8 to 8:1 w/w ratio. In some embodiments, the composition described herein comprises Tryptophol derivative or 4-Ethyl Phenol derivative in 1:7 to 7:1 w/w ratio. In some embodiments, the composition described herein comprises Tryptophol derivative or 4-Ethyl Phenol derivative in 1:6 to 6:1 w/w ratio. In some embodiments, the composition described herein comprises Tryptophol derivative or 4-Ethyl Phenol derivative in 1:5 to 5:1 w/w ratio. In some embodiments, the composition described herein comprises Tryptophol derivative or 4-Ethyl Phenol derivative in 1:4 to 4:1 w/w ratio. In some embodiments, the composition described herein comprises Tryptophol derivative or 4-Ethyl Phenol derivative in 1:3 to 3:1 w/w ratio. In some embodiments, the composition described herein comprises Tryptophol derivative or 4-Ethyl Phenol derivative in 1:2 to 2:1 w/w ratio. In some embodiments, the composition described herein comprises Tryptophol derivative or 4-Ethyl Phenol derivative in 1:1 to 1:1 w/w ratio. Each possibility represents a separate embodiment of the present invention.

In some embodiments, Tryptophol derivative or 4-Ethyl Phenol derivative are present at a concentration of at least 1 µM, at least 2 µM, at least 5 pM, at least 10 µM, at least 15 µM, at least 20 µM, at least 30 µM, at least 40 µM, at least 50 µM, at least 75 µM, at least 100 µM, at least 150 µM, at least 200 µM, at least 225 µM, or at least 350 µM, within the composition, or any value and range therebetween. Each possibility represents a separate embodiment of the invention. In some embodiments, Tryptophol derivative or 4-Ethyl Phenol derivative are present at a concentration of 1-10 µM, 5-20 µM, 10-30 µM, 20-40 µM, 25-50 µM, 30-60 µM, 40-70 µM, 50-80 µM, 65-90 µM, 70-100 µM, 80-110 µM, 90-120 µM, 110-160 µM, 150-275 µM, or 250-500 µM within the composition. Each possibility represents a separate embodiment of the invention.

In some embodiments, the composition or the microorganism mixture comprise each of Tryptophol derivative and 4-Ethyl Phenol derivative at a concentration of at least 50 nM, at least 100 nM, at least 1 µM, at least 2 µM, at least 5 µM, at least 10 µM, at least 15 µM, at least 20 µM, at least 30 µM, at least 40 µM, at least 50 µM, at least 75 µM, at least 100 µM, at least 150 µM, at least 200 µM, at least 225 µM, or at least 350 µM, within the composition, or any value and range therebetween. Each possibility represents a separate embodiment of the invention. In some embodiments, the composition or the microorganism mixture comprise each of Tryptophol derivative and 4-Ethyl Phenol derivative at a concentration of 10-50 nM, 40-100 nM, 75-500 nM, 450-900 nM, 0.75-1.5 µM, 1-10 µM, 5-20 µM, 10-30 µM, 20-40 µM, 25-50 µM, 30-60 µM, 40-70 µM, 50-80 µM, 65-90 µM, 70-100 µM, 80-110 µM, 90-120 µM, 110-160 µM, 150-275 µM, or 250-500 µM within the composition. Each possibility represents a separate embodiment of the invention.

As defined herein, the term "half maximal inhibitory concentration (IC₅₀)" refers to a measure of the potency of a substance in inhibiting a specific biological or biochemical function. In some embodiments, a composition (e.g., a pharmaceutical composition) comprising a tryptophol derivative, a 4-Ethyl Phenol derivative, or any combination thereof, has an IC₅₀ at the micromolar level. In some embodiments, a composition comprising a microorganism mixture as disclosed herein, has an IC₅₀ at the micromolar level. In some embodiments, micromolar level comprises 1,000 µM at most, 900 µM at most, 800 µM at most, 700 µM at most, 600 µM at most, 500 µM at most, 400 µM at most, 300 µM at most, 200 µM at most, 100 µM at most, 75 µM at most, 50 µM at most, 35 µM at most, 20 µM at most, 15 µM at most, 10 µM at most, 5 µM at most, or 1 µM at most, or any value and range therebetween. Each possibility represents a separate embodiment of the invention. In some embodiments, micromolar level comprises 1-10 µM, 5-20 µM, 15-30 µM, 25-500 µM, 40-75 µM, 70-120 µM, 100-200 µM, 150-300 µM, 250-400 µM, 375-500 µM, 400-650 µM, 600-850 µM, or 800-1,000 µM. Each possibility represents a separate embodiment of the invention.

As used herein, the term "carrier" refers to a diluent, adjuvant, excipient, or vehicle with which the active compound is administered. Such carriers can be sterile liquids, such as water-based and oils, including those of petroleum, animal, vegetable or synthetic origin, such as peanut oil, soybean oil, mineral oil, sesame oil and the like, polyethylene glycols, glycerin, propylene glycol or other synthetic solvents.

Water may be used as a carrier such as when the active compound is comprised by a pharmaceutical composition being administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like. The composition, if desired, can also contain minor amounts of wetting or emulsifying agents, or pH buffering agents such as acetates, citrates or phosphates. Antibacterial agents such as benzyl alcohol or methyl parabens; antioxidants such as ascorbic acid or sodium bisulfite; and agents for the adjustment of tonicity such as sodium chloride or dextrose are also envisioned. The carrier may comprise, in total, from about 0.1% to about 99.99999% by weight of the compositions presented herein.

An embodiment of the invention relates to molecules of the present invention, presented in unit dosage form and prepared by any of the methods well known in the art of pharmacy. In one embodiment, the unit dosage form is in the form of a tablet, capsule, lozenge, wafer, patch, ampoule, vial or pre-filled syringe.

In addition, in vitro assays may optionally be employed to help identify optimal dosage ranges. The precise dose to be employed in the formulation will also depend on the route of administration, and the nature of the disease or disorder, and should be decided according to the judgment of the practitioner and each patient's circumstances. Effective doses can be extrapolated from dose-response curves derived from in-vitro or in-vivo animal model test bioassays or systems.

In one embodiment, the composition of the present invention is administered in the form of a pharmaceutical composition comprising the two active components of this invention together with a pharmaceutically acceptable carrier or diluent. In another embodiment, the composition of the invention can be administered in any conventional oral, parenteral or transdermal dosage form.

As used herein, the terms "administering", "administration", and like terms refer to any method which, in sound medical practice, delivers a composition containing an active agent to a subject in such a manner as to provide a therapeutic effect.

In some embodiments, the pharmaceutical composition comprising the compound of the invention, or any derivative or combination thereof, or the microorganism mixture of the invention, is administered via oral (i.e., enteral), rectal, vaginal, topical, nasal, ophthalmic, transdermal, subcutaneous, intramuscular, intraperitoneal or intravenous routes of administration. The route of administration of the pharmaceutical composition will depend on the disease or condition to be treated. Suitable routes of administration include, but are not limited to, parenteral injections, e.g., intradermal, intravenous, intramuscular, intralesional, subcutaneous, intrathecal, and any other mode of injection as known in the art. In addition, it may be desirable to introduce the pharmaceutical composition of the invention by any suitable route, including intraventricular and intrathecal injection; intraventricular injection may be facilitated by an intraventricular catheter, for example, attached to a reservoir. Pulmonary administration can also be employed, e.g., by use of an inhaler or nebulizer.

For topical application, the compound of the invention, or any derivative or combination thereof, or the microorganism mixture of the invention, can be combined with a pharmaceutically acceptable carrier so that an effective dosage is delivered, based on the desired activity. The carrier can be in the form of, for example, and not by way of limitation, an ointment, cream, gel, paste, foam, aerosol, suppository, pad or gelled stick.

For oral applications, the pharmaceutical composition may be in the form of tablets or capsules, which can contain any of the following ingredients, or compounds of a similar nature: a binder such as microcrystalline cellulose, gum tragacanth or gelatin; an excipient such as starch or lactose; a disintegrating agent such as alginic acid, Primogel, or corn starch; a lubricant such as magnesium stearate; or a glidant such as colloidal silicon dioxide. When the dosage unit form is a capsule, it can contain, in addition to materials of the above type, a liquid carrier such as fatty oil. In addition, dosage unit forms can contain various other materials which modify the physical form of the dosage unit, for example, coatings of sugar, shellac, or other enteric agents. The tablets of the invention can further be film coated. In some embodiment, oral application of the pharmaceutical composition may be in the form of drinkable liquid. In some embodiment, oral application of the pharmaceutical composition may be in the form of an edible product. Non-limiting examples for oral carriers include, but are not limited to: milk, yogurt, probiotic yogurt, kefir, fermented milk or others.

For purposes of parenteral administration, solutions in sesame or peanut oil or in aqueous propylene glycol can be employed, as well as sterile aqueous solutions of the corresponding water-soluble salts. Such aqueous solutions may be suitably buffered, if necessary, and the liquid diluent first rendered isotonic with sufficient saline or glucose. These aqueous solutions are especially suitable for intravenous, intramuscular, subcutaneous and intraperitoneal injection purposes.

The compositions also include incorporation of the active material into or onto particulate preparations of polymeric compounds such as polylactic acid, polglycolic acid, hydrogels, etc., or onto liposomes, microemulsions, micelles, unilamellar or multilamellar vesicles, erythrocyte ghosts, or spheroplasts. Such compositions will influence the physical state, solubility, stability, rate of in vivo release, and rate of in vivo clearance.

In one embodiment, it will be appreciated that the molecules of the present invention, or any derivative or combination thereof, or the microorganism mixture of the invention, can be provided to the individual with additional active agents to achieve an improved therapeutic effect as compared to treatment with each agent by itself. In another embodiment, measures (e.g., dosing and selection of the complementary agent) are taken to adverse side effects which are associated with combination therapies.

In one embodiment, depending on the severity and responsiveness of the condition to be treated, dosing can be of a single or a plurality of administrations, with course of treatment lasting from several days to several weeks or until cure is affected or diminution of the disease state is achieved.

In some embodiments, the composition of the preset invention is administered in a therapeutically safe and effective amount. As used herein, the term "safe and effective amount" refers to the quantity of a component which is sufficient to yield a desired therapeutic response without undue adverse side effects (such as toxicity, irritation, or allergic response) commensurate with a reasonable benefit/risk ratio when used in the presently described manner. In another embodiment, a therapeutically effective amount of the molecules, or any derivative or combination thereof, is the amount of the mentioned herein molecules necessary for the in vivo measurable expected biological effect. The actual amount administered, and the rate and time-course of administration, will depend on the nature and severity of the condition being treated. Prescription of treatment, e.g. decisions on dosage, timing, etc., is within the responsibility of general practitioners or specialists, and typically takes account of the disorder to be treated, the condition of the individual patient, the site of delivery, the method of administration and other factors known to practitioners. Examples of techniques and protocols can be found in Remington: The Science and Practice of Pharmacy, 21st Ed., Lippincott Williams & Wilkins, Philadelphia, Pa., (2005). In some embodiments, preparation of effective amount or dose can be estimated initially from in vitro assays. In one embodiment, a dose can be formulated in animal models and such information can be used to more accurately determine useful doses in humans.

In one embodiment, toxicity and therapeutic efficacy of the active ingredients described herein can be determined by standard pharmaceutical procedures in vitro, in cell cultures or experimental animals. In one embodiment, the data obtained from these in vitro and cell culture assays and animal studies can be used in formulating a range of dosage for use in human. In one embodiment, the dosages vary depending upon the dosage form employed and the route of administration utilized. In one embodiment, the exact formulation, route of administration and dosage can be chosen by the individual physician in view of the patient's condition. [See e.g., Fingl, et al., (1975) "The Pharmacological Basis of Therapeutics", Ch. 1 p.1].

Pharmaceutical compositions containing the molecules of the present invention, or any derivative or combination thereof, or the microorganism mixture of the present invention, as the active ingredient can be prepared according to conventional pharmaceutical compounding techniques. See, for example, Remington's Pharmaceutical Sciences, 18th Ed., Mack Publishing Co., Easton, Pa. (1990). See also, Remington: The Science and Practice of Pharmacy, 21st Ed., Lippincott Williams & Wilkins, Philadelphia, Pa. (2005).

In one embodiment, compositions including the preparation of the present invention formulated in a compatible pharmaceutical carrier are prepared, placed in an appropriate container, and labeled for treatment of an indicated condition.

In one embodiment, compositions of the present invention are presented in a pack or dispenser device, such as an FDA approved kit, which contains, one or more unit dosages forms containing the active ingredient. In one embodiment, the pack, for example, comprises metal or plastic foil, such as a blister pack. In one embodiment, the pack or dispenser device is accompanied by instructions for administration. In one embodiment, the pack or dispenser is accommodated by a notice associated with the container in a form prescribed by a governmental agency regulating the manufacture, use or sale of pharmaceuticals, which notice is reflective of approval by the agency of the form of the compositions or human or veterinary administration. Such notice, in one embodiment, is labeling approved by the U.S. Food and Drug Administration for prescription drugs or of an approved product insert.

Any concentration ranges, percentage range, or ratio range recited herein are to be understood to include concentrations, percentages or ratios of any integer within that range and fractions thereof, such as one tenth and one hundredth of an integer, unless otherwise indicated.

Any number range recited herein relating to any physical feature, such as polymer subunits, size or thickness, are to be understood to include any integer within the recited range, unless otherwise indicated.

In the discussion unless otherwise stated, adjectives such as "substantially" and "about" modifying a condition or relationship characteristic of a feature or features of an embodiment of the invention, are understood to mean that the condition or characteristic is defined to within tolerances that are acceptable for operation of the embodiment for an application for which it is intended. Unless otherwise indicated, the word "or" in the specification and claims is considered to be the inclusive "or" rather than the exclusive or, and indicates at least one of, or any combination of items it conjoins.

It should be understood that the terms "a" and "an" as used above and elsewhere herein refer to "one or more" of the enumerated components. It will be clear to one of ordinary skill in the art that the use of the singular includes the plural unless specifically stated otherwise. Therefore, the terms "a", "an" and "at least one" are used interchangeably in this application.

For purposes of better understanding the present teachings and in no way limiting the scope of the teachings, unless otherwise indicated, all numbers expressing quantities, percentages or proportions, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained. At the very least, each numerical parameter should at least be construed in light of the number of reported significant digits and by applying ordinary rounding techniques.

In the description and claims of the present application, each of the verbs, "comprise," "include" and "have" and conjugates thereof, are used to indicate that the object or objects of the verb are not necessarily a complete listing of components, elements or parts of the subject or subjects of the verb.

Other terms as used herein are meant to be defined by their well-known meanings in the art.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination or as suitable in any other described embodiment of the invention. Certain features described in the context of various embodiments are not to be considered essential features of those embodiments, unless the embodiment is inoperative without those elements.

### EXAMPLES

Generally, the nomenclature used herein, and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds.) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Strategies for Protein Purification and Characterization - A Laboratory Course Manual" CSHL Press (1996). Other general references are provided throughout this document.

### Materials and Methods

### Sample and culture of probiotic yogurt (e.g., kefir)

Fifty (50) gr of kefir grains were inoculated in a 1,000 ml flask containing 800 ml of pasteurized cow milk (TARA, Israel) and covered with sterile gauze. The mixture was subsequently cultivated at 28 °C for 24 h. Subsequently the probiotic yogurt was filtered to separate the grains from the fermented milk. The cultured microorganisms in the fermented milk were taken to the sequence analysis.

DNA was extracted from the probiotic yogurt (e.g., kefir) cultures using a PowerSoil^{®} DNA Isolation Kit (MoBio Laboratories, Loker Avenue West Carlsbad; CA), according to the manufacturer's instructions. Total DNA was sent to DNA Services (DNAS) Facility, at the Research Resources Center, the University of Illinois at Chicago (UIC) for shotgun sequencing. Sequences method Nextera XT with sequence of paired - end 2×150 reads illumine Nex-Keg 500 sequencer was performed. The reads were uploaded as FASTQ to One Codex website and were analyzed for metagenomics taxonomic annotation results.

### Imaging flow cytometry

The probiotic yogurt (e.g., kefir) was diluted 1:10 with water and analyzed by ImageStreamX Mk II (Amnis Corporation, Seattle, WA, USA.). A default mask of the (Bright field) BF was used to identify the microorganisms in focus and plot the Gradient root mean square (RMS). Population analyses were carried out according to Infra-red (IR) absorbance intensities that were detected from the bacterial cells, but not from the fungal cells. For the purpose of calculating the count values a custom mask was applied to the fungal cells on the auto fluorescence channel. Then, according to the intensity values calculated by the instrument, the microorganisms were divided into subpopulations.

### Extraction of probiotic yogurt-derived organic molecules

The produced probiotic yogurt (e.g., kefir) was centrifuged at 4,000 rpm for 10 min to separate the sediment and supernatant. Five hundred (500) ml of the supernatant were then moved to separating funnel and mixed with 500 ml of Ethyl acetate. The organic phase was separated from the supernatant and moved to a round bottom flask to evaporate all the solvent until a solid residue was obtained, containing the organic molecules from the probiotic yogurt.

### Bioactivity of extracted organic molecules

Bioluminescence assay was performed with mutant bacteria lacking the nucleotide sequences encoding the relevant autoinducers (AI). Additionally, the bacteria were cloned with bioluminescence reporter plasmids in order to measure their luminescence intensity when quorum sensing gene expression occurs. Ninety-six (96) well microtiter plate assays were used to assess AI activation (agonist) or inhibition (antagonist) of the following bacteria: Agrobacterium tumefaciens, which responds to the C8 autoinducer, and both Vibrio cholerae and Vibrio harveyi, which respond to the (S)-4,5-dihydroxy-2,3-pentanedione (DPD) autoinducer to communicate.

Agrobacterium tumefaciens A136 pCF218 pMV26 culture was grown in Luria-Bertani broth (LB; Miller's broth) supplemented with 25 µg/ml of kanamycin and 4.5 µg/ml of tetracycline at 28-30 °C for 24 h. Both Vibrio harveyi and Vibrio cholerae strains were grown in LB Broth (Lennox) supplemented with tetracycline at 30 °C for 24 h. The overnight cultures were diluted to an absorbance density (OD₆₀₀) of 0.05 by fresh LB medium. The probiotic yogurt (e.g., kefir)-extracted molecules were tested at gradient concentration in two types of experiments: competition assay in the absence (activation/agonist) or presence (inhibition/antagonist) of 400 pM 3-oxo-C8-HSL (A. tumefaciens A136 pCF218 pMV26) CAI1 (Vibrio Cholerae MM920) 200 nM DPD (Vibrio harveyi MM30) and the OD₆₀₀ of 0.05 by fresh LB medium. A clear bottom 96-well microliter plate was prepared with wells containing test compounds serially diluted into the LB medium (triplicates). A total of 100 µl of the diluted cells were added to each well. The control sample contained the bacteria and specific AI molecules without the yogurt extracted molecules. Luminescence was measured every 20 min for 19 h with continuous shaking at 30 °C, using a Microtiter Plate Reader (Varioskan Flash, Thermo). Average luminescence values divided by OD₆₀₀ values were plotted against the added compound concentration.

### Extraction of Kluyveromyces marxianus-derived organic molecules

K. marxianus strain HA 63 [NRRL Y-8281, CBS 712] was cultured in 300 ml of yeast and malt LB and grown at 28 °C for 24 h and agitation at 100 rpm. The culture was centrifuged at 4,000 rpm for 10 min, and the supernatant was collected. The supernatant was then moved to a separating funnel and mixed with an equivalent volume of Ethyl acetate for the extraction. The blend was shaken for 10 min, the organic phase was transferred to a new tube, and the extraction was repeated three times. Next, the extracts were evaporated to remove all fluids and finally dried at the lyophilizer to avoid water presence.

### Liquid chromatography-Mass spectrometry (LC-MS)

K. marxianus crude extract was injected to LC-MS to identify metabolites originating from the yeast. The chromatograph of the K. marxianus crude extract was compared to that of a medium crude extract in order to identify molecules specifically linked to K. marxianus metabolism. Molecules molecular weights were determined by MS using an LTQ XL Orbitrap with a static nano-spray in positive ion mode (Waters Acquity QDA with PDA and QDA detectors).

### Anti-biofilm activity of synthesized molecule (203)

For static biofilm assay, overnight cultures of P. aeruginosa (PA01), Salmonella and Staphylococcus aureus strains were diluted 1:10 in fresh LB medium containing a final concentration of synthesized molecule (203) of 20 or 50 µM, or DMSO (up to 1%) as control. Biofilms were grown under non-shaking conditions at 37 °C in 96-well plate (Thermo Scientific, Rochester, NY, USA). The usage of the synthesized molecule (203) did not impact cell growth, by visualizing viable cells stained green and dead cells stained red with the BacLight^{®} Dead/Live Kit (Invitrogen, Eugene, OR, USA). No increase in dead cells was observed in presence of synthesized molecule (203) compared to control biofilm. The stained cells were washed twice with PBS. Biofilm images were taken by CLSM (Plan-Apochromat 20×/0.8 M27, Zeiss LSM880, Germany).

### Biofilm modulation activity of synthesized Tryptophol acetate and Tyrosol acetate

The various pathogenic indicator strains used in this study were V. cholerae strain MM920 (ΔCqsA ΔluxQ), *Vibrio harveyi* strain MM30 (LuxS, Tn5). To initiate biofilm formation, bacteria was grown in the absence of the *V. cholerae* autoinducer 1 (CAI1). In addition, the bacteria were grown in presence of: 900 nM CAI1 (Vibrio Cholerae MM920), 900 nM CAI1, 200 µM Tryptophol acetate and 200 µM Tyrosol acetate, and 200 µM Tryptophol acetate and 200 µM Tyrosol acetate.

*Salmonella Enteritidis* was provided. *Staphylococcus aureus* group IV strain, RN8242. *Pseudomonas aeruginosa* PA01, *Salmonella* and *Staphylococcus aureus* strains were incubated for 24 h in LB broth medium at 37 °C.

Biofilms were grown under shaking conditions at 30 °C in 96-well plate (Thermo Scientific, Rochester, NY, USA). The usage of the synthesized molecules did not impact cell growth, visualized as described above. The stained cells were washed twice with PBS. Biofilm images were taken by CLSM (Plan-Apochromat 20×/0.8 M27, Zeiss LSM880, Germany).

### Antifungal activity

Antifungal activity was estimated using three types of fungi (Sclerotinia, Botrytis, and Penicillium). Fungi were grown in Potato Dextrose Broth (PDB) medium by taking a piece of agar from a plate on which the fungus was found into a Falcon containing 30 ml PDB and incubated at 22 °C until it produced mycelium. The fungal suspension was diluted 1:1 with fresh PDB and incubated at 22 °C for ~48 hours with 75 µl of probiotic yogurt (e.g., kefir) crude extract dissolved in DMSO, or with DMSO as a control (refreshment sample). After 48 hours the fungal suspension was filtered and 10 µl of the filtrate was transferred to the center of a Potato Dextrose Agar (PDA) plate containing either 1.5% (v/v) of probiotic yogurt crude extract or DMSO as a control.

### Chemical synthesis of Tyrosol acetate and Tryptophol acetate

Homovanillyl alcohol (1 mmol), was dissolved in 3 ml of dichloromethane (DCM), then 1.2 mmol of acetyl chloride was added to the reaction mixture and allowed to stir for 24 h at room temperature until disappearance of the substrate. After completion of the reaction the DCM was evaporated under vacuum conditions and the mixture solubilized with ethyl acetate and washed with a saturated solution of NaCl. The aqueous phase was extracted with ethyl acetate (3 × 50 mL) and washed with 50 mL saturated NaCl, and then dried over magnesium sulfate. Purification by flash chromatography on silica gel using EtOAc-hexanes (2:1) for elution provided Tyrosol acetate (2%, 18 mg). Rf 0.43 (30% EtOAc-70% hexanes); validated by LC-MS and 1H NMR.

3-(2-Hydroxyethyl)indole (3.224 gr, 20 mmol) was dissolved in 40 mL pyridine and 2,648 µL (28 mmol) acetic anhydride was added dropwise and allowed to stir for 15 h at room temperature. The solution was poured into 160 mL H₂O solution and stirred for 20 min. The heterogeneous mixture was separated, and the aqueous layer was extracted with CH₂Cl₂ (4 × 40 mL). The combined organic layers were washed with brine, dried (MgSO₄), filtered through celite and concentrated under reduced pressure. Purification by flash chromatography on silica gel using EtOAc/hexanes for elution provided Tryptophol acetate as colorless oil (5.2%, 0.212 gr). Rf 0.46 (50% EtOAc/hexanes); validated by LC-MS and 1H NMR.

### Determination of quorum sensing activity

The effect of the Kefir biomass crude extract molecules on the following bacteria, A. *tumefaciens* A136, *V. cholerae* MM920 and V. *harveyi* MM30 were measured as described by Brenier et al. (2008). The overnight culture was diluted to an absorbance density (OD₆₀₀) of 0.05 by fresh LB medium. A clear bottom 96-well microliter plate was prepared with wells containing test compounds serially diluted into the LB medium (triplicates). A total of 100 µL of the diluted cells was added to each well. The control sample contained the bacteria and specific autoinducer molecules without the kefir crude extracted molecules. Luminescence was measured every 20 min for 19 h with continuous shaking at 30 °C, using a Microtiter Plate Reader (Varioskan Flash, Thermo). Two types of experiments were performed: a competition assay in the presence of 400 pM 3-oxo-C8-HSL (*A. tumefaciens* A136), CAI-1 (*V. Cholerae* MM920) 200 nM DPD (V. *harveyi* MM30) and the other in the absence of these AI's to measure agonistic activity of the crude extracted molecules. Average luminescence values divided by OD₆₀₀ values were plotted against the added compound concentration.

### Biofilm modulation activity of kefir biomass crude extract molecules

*P. aeruginosa* PA01, *Salmonella* and *Staphylococcus aureus* strains were incubated for 24 hr at 37 °C. The bacterial suspension was diluted 1:10 with fresh LB and incubated for 3 h with the kefir biomass crude extract molecules or with DMSO as a control (refreshment sample). In all cultures, DMSO concentration was up to 1% (v/v). 200 µl of the broth medium mix with yogurt extract or with DMSO as control were placed in each well of a 96-well plate (Thermo Scientific, Rochester, NY, USA). 2 µl refreshment samples were added to each suitable well. The plate incubated for 24 hr at 37 °C. After the incubation the samples washed three times with PBS. For visualizing of viable cells, the bacteria were stained green and dead cells stained red using the BacLight^{®} Dead/Live Kit (Invitrogen, Eugene, OR, USA). The stained cells were washed twice with PBS. Biofilm images were taken by CLSM (Olympus, Tokyo, Japan). Image processing was done using IMARIS software (Bitplane, Zurich, Switzerland).

### Biofilm modulation activity of synthesized Tryptophol acetate and Tyrosol acetate

For static biofilm assay, overnight cultures of *V. cholerae* strain MM920 was diluted 1:10 in fresh LB medium containing a final concentration of 100 µM from each Tryptophol acetate and Tyrosol acetate or DMSO (up to 1%) as control. The inventors analyzed two type of samples. One in absence of the *V. cholerae* autoinducer (CAI-1) and the other one in presence of 900 nM CAI-1. Biofilms were grown under non-shaking conditions at 37 °C in 96-well plate (Thermo Scientific, Rochester, NY, USA). The usage of the synthesized molecules did not impact cell growth, by visualizing viable cells stained green and dead cells stained red with the BacLight^{®} Dead/Live Kit (Invitrogen, Eugene, OR, USA). No increase in dead cells was observed in presence of both synthesized molecules compared to control biofilm. The stained cells were washed twice with PBS. Biofilm images were taken by CLSM (Plan-Apochromat 20×/0.8 M27, Zeiss LSM880, Germany).

### Nuclear magnetic resonance (NMR)

Spectra for ¹H and ¹³C nuclear magnetic resonance (NMR) were recorded at room temperature (RT) with a Bruker Avance DPX500 instrument (500 MHz).

### Quantitative Real-time PCR (RT-PCR) analysis

RNA was extracted from wild-type strain VC1 cultured in LB lennox medium supplemented with or without 100 µM Tryptophol acetate or Tyrosol acetate grown to an A600 of approximately 1.0_{OD} using the RNA Protect Bacteria reagent and the RNeasy^{®} Mini Kit (Qiagen, Valencia, CA) as per the manufacturer's instructions, including the on-column DNase I digestion described by the manufacturer. Purified RNA was quantified using a Banalyzer (Eppendorf, Hamburg, Germany). cDNA was synthesized from 1 µg of RNA using PrimeScriptTM RT reagent kit (Takara, Ohtsu, Japan). The reaction was incubated at 37 °C for 30 min, and 2 µl of cDNA was subjected to RT-PCR analysis on an AB Step One Plus PCR system (Applied Biosystems, Carlsbad, CA), using qPCRBIO SyGreen Blue mix Hi-ROX (PCR Biosystems, London, UK). RT-PCR was performed in a 96-well plate (Bio-Rad) in triplicate in a 20-µl volume. The mdh gene was used as an endogenous loading control for the reactions. The amount of transcript was analyzed with StepOnePlus Software V2.3 (Applied Biosystems Carlsbad, CA). Mdh forward primer 5'-CTGGCGGCATTGGTCAAGCCC-3' (SEQ ID NO: 1); Mdh reverse primer 5'-ACCCGGTGTGACAGGCGCAA-3' (SEQ ID NO: 2); vpsT forward primer 5'-CGCAGTATTCAGATGCTGGTG-3' (SEQ ID NO: 3); vpsT reverse primer 5'-GACCTCTTTCGCATCAGGACA-3' (SEQ ID NO: 4); ctxA forward primer 5'-AGCAGTCAGGTGGTCTTATGC-3' (SEQ ID NO: 5); ctxA reverse primer 5'-CCCGTCTGAGTTCCTCTTGC-3' (SEQ ID NO: 6); aphA forward primer 5'-ACCGGGTACGATATAACCAAAGAG-3' (SEQ ID NO: 7); aphA reverse primer 5'-GATGGCTGGCTTTCCAGAAG-3' (SEQ ID NO: 8). For Transcriptome libraries construction, we used the purified RNA as described in section Quantitative Real-time PCR (RT-PCR) Analysis. The ribosomal RNA was depleted using MICROBExpress kit (Invitrogen by Thermo Fisher Scientific, Lithuania). The libraries preparation was performed using SMARTer Standard RNA-Seq kit (Takara Bio, USA) according to manufacture instruction. High Throughput Sequencing Analysis was done by Illumina HiSeq 2500, SR 50 bp and barcode Raw data quality values phred+33. the amount and percentage of reads that passed the sequencer's automated quality filter, control mapping value, error rate, and per-base quality scores indicate that the sequencing was of high quality. Only a small percentage of reads were discarded due to trimming and the peak of the length distribution is at the original length of 51. The Software and applications that were used for comparison analyses are Library quality control: FASTQC version 0.11.5; Trim_galore (uses cutadapt version 1.10); Mapping: Tophat2 version 2.1.0 (uses Bowtie2 version 2.2.6); Gene counting: HTseq-count version 0.6.1. For normalization and differential expression analysis, DESeq2 R package version 1.18.1 Input files. The reference genome used in the analysis was of Vibrio cholerae O1 biovar El Tor str. N16961, from the following link: ftp://ftp.ncbi.nlm.nih.gov/genomes/all/GCF/000/006/745/GCF_000006745.1_ASM674 v1. Counting was performed using an annotation file downloaded at the following link: ftp://ftp.ncbi.nlm.nih.gov/genomes/all/GCF/000/006/745/GCF_000006745.1_ASM674 v1/G CF_000006745.1_ASM674v1_genomic.gtf.gz. All these files can be found in our FTP server, see section 4- Analysis result files.

### GM1-enzyme-linked immunosorbent assay (ELISA) - Cholerae Toxin Detection Assay

GM1 (monosialotetrahexosylganglioside) was seeded and immobilized on a 96-well white/clear bottom microtiter plate (Greiner) microtiter plates with the following procedure: GM1 stock solution (2 mg/mL in PBS) was diluted with PBS (final conc. 10 µg/mL). 200 µL of the GM1 solution was added to each well and incubated at 37 °C without shaking for 4-16 hours. The plates were washed with PBS (x3). Bovine serum albumin (BSA) was dissolved in PBS (final conc. 4 mg/mL) and 200 µL of the BSA-PBS were added to each well and incubated at 37 °C without shaking for at least 4 hours. The plates were washed with PBS (x3) and were stored in fridge until use. VC1 wild-type strain with of the tested compounds (Tryptophol acetate and Tyrosol acetate both in concentration of 100 µM ) and a control with bacteria only, were grown and incubated with aeration and shaking overnight in LB medium at 30 °C. The cultures were spinned-down for 5 min at 5,000 rcf and the supernatant was taken and diluted 1:2 with a BSA-PBS 4 mg/mL solution. 200 µL of the diluted supernatant was added in six-replicates to each GM1 coated well and the plate was incubated at 37 °C with gentle shaking for at least 30 min. Meanwhile, rabbit antitoxin serum (RSA) stock solution was diluted 1:999 with BSA-PBS. The plates were washed with PBS (×3), 200 µL of the RSA solution were added to each well and the plate was incubated at 37 °C with gentle shaking for at least 30 min. Meanwhile, goat anti-rabbit immunoglobulin G (IgG) H&L alkaline phosphatase stock solution (1 mg/mL in DDW) was diluted 1:1,499 with BSA-PBS. The plates were washed with PBS (×3), 200 µL of the IgG solution were added to each well and the plate was incubated at 37 °C with gentle shaking for at least 30 min. Meanwhile luminol working solution was prepared by making two stock solutions. Stock A was prepared by adding 0.1 mL luminol 250 mM in DMSO, 44 µL coumaric acid 90 mM in DMSO, 1 mL tris-HCl 1 M pH=8.5, and DDW up to a final volume of 10 ml. Stock B was prepared by adding 6.4 µl hydrogen peroxide 30%, 1 ml Tris-HCl 1 M pH=8.5, and DDW up to a final volume of 10 ml. The plates were washed with PBS (×3) and quickly equal volumes of stock A and B were mixed making the luminol working solution. 100 µL of the luminol working solution were added to each well, the plate was shaken for 1.5-2 minutes and the luminescence was measured using a Microtiter Plate Reader (Varioskan Flash, Thermo). Luminescence values were normalized to the control.

### Macrophages cell collection

Peritoneal macrophages were harvested using the protocol as described previously (Zhang et al., 2008). Elicited macrophages were harvested from WT mice which had received a 2.5 ml intraperitoneal administration of 3% Brewer thioglycolate broth (Sigma, UK) 72 hr prior collection.

### In vitro LPS stimulation

Peritoneal macrophages cells (5 × 10⁵/ml) were cultured with 0.1 µg/ml Lipopolysaccharides (LPS *-Escherichia coli* serotype 0127:B8, Sigma-Aldrich) and in the presence of 100 µM: tryptophol acetate, tyrosol acetate, dopamine HCl, caffeic acid, or combination of tryptophol acetate and tyrosol acetate, for 24 hr. Following culture, supernatants were collected and stored at -20 °C for the detection of the IL-6 cytokine. For IL-1α detection, the inventors induced macrophages lysis by adding fresh RPMI medium and thawing three times.

### Cytokine ELISA

IL-1α and IL-6 levels were measured in peritoneal exudate fluids and LPS-stimulated culture supernatants by ELISA. To detect the cytokines, a R&D ELISA kit (Catalog # MLA00 and DY406 , respectively) was used as per the manufacturer's instructions. A standard curve was constructed using serial dilutions of purified IL-1α or IL-6 starting at a concentration of 15 ng/ml.

### Thioflavin T (ThT) assay

ThT fluorescence measurements were taken at 37 °C using 96-well black plate on a Biotek Synergy H1 plate reader. Measurements were made on a samples containing 30 µM amyloid beta [aβ(1-42)] in the absence or presence of tryptophol acetate in different concentrations. A 120-µL aliquot of the aggregation reaction was mixed with 10 µM ThT in phosphate buffer (pH 7.4). The fluorescence intensity was measured every 30 min for 24 hr at λ excitation (λex) = 440 and λ emission (λem) = 485 nm.

### Surface plasmin resonance (SPR)

SPR sensor chip with Oligomer A11 antibody, an anti-amyloid oligomer conformation-specific antibody was tested with samples containing 30 µM aβ(1-42) in the absence or presence of tryptophol acetate in different concentrations.

### Transmission electron microscopy (TEM)

TEM grids were prepared after 24 hr incubation at 37 °C of samples containing 30 µM aβ(1-42) in the absence or presence of tryptophol acetate in different concentrations.

### EXAMPLE 1

### Identification of microorganisms in a probiotic yogurt (e.g., kefir)

The inventors found that the major bacterial species within the probiotic yogurt were Lactobacillus kefiranofaciens, Lactobacillus, Lactococcus lactis, Propionibacterium, and Leuconostoc mesenteroides. There were many other genera present in the probiotic yogurt; however, they typically represented less than 0.5% of the microflora. A significant finding in the sequencing analysis, and different than other reported probiotic yogurts (such as kefirs), was the identification of the probiotic yeast Kluyveromyces marxianus which was found to be a dominant species in the disclosed probiotic yogurt (59.04%) (**Fig. 1A**). The morphology of this yeast in the probiotic yogurt was found to be similar to its monoculture morphology (**Figs. 1B and 1C**).

### EXAMPLE 2

### Composition of microorganism subpopulation in a probiotic yogurt (e.g., kefir)

The inventors obtained scatterplot of auto fluorescence intensity vs. BF detail intensity of population R5 (19,539 events; **Fig. 2A**), as summarized in the table below. Distinct subpopulations R3, R6, and R7 were visible on the plot (Fig. 2A), as well as other composite subpopulations (R8). Different colors selected to different gates corresponded to sets of images displayed at the periphery of the plot. R3 (orange dots; **Fig. 2A**) were the populations defined as bacteria cells (86.1%; **Fig. 2B**), R6 (light blue dots; **Fig. 2A**) were the populations defined as fungal cells (4.94%; **Fig. 2C**) and R7 (light yellow dots; **Fig. 2A**) were the populations defined as aggregation of both fungal and bacteria (7.58%; **Fig. 2D**). R8 (maroon dots; **Fig. 2A**) population were larger aggregates (**Fig. 2E**) and therefore were defined as a sub-population of R7.

| | **Population** | **Count** | **% Gated** |
|---|---|---|---|
| All | R5 & R1 | 19,539 | 100 |
| Bacteria only | R3 & R5 & R1 | 16,832 | 86.1 |
| Fungi only | R6 & R5 & R1 | 966 | 4.94 |
| Aggregation of Bacteria & Fungi | R7 & R5 & R1 | 1,482 | 7.58 |
| | R8 & R5 & R1 | 100 | 0.51 |

### EXAMPLE 3

### Characterization and mass spectrometric (MS) analysis of crude extracts

A K. marxianus monoculture crude extract was injected to LC-MS to identify molecules which originated from the yeast's metabolism. The chromatograph of the crude extract was compared to medium crude extract to examine which molecules were specifically connected to K. marxianus metabolism. Two molecules were identified only in the K. marxianus crude extract chromatogram (**Fig. 4B**) and not in the medium extract chromatogram (**Fig. 4A**), with retention time (RT) of 11.68 and 13.84 min. The molecular weights were determined by MS and were dominated by m/z peaks of 180 and 203, respectively. Furthermore, both peaks were observed in a LC-MS chromatogram of a crude extract of a probiotic yogurt (e.g., kefir) comprising the K. marxianus (**Fig**. **3B**). These peaks were not observed in the extract of the milk which was used in fermentation for producing the probiotic yogurt (**Fig**. **3A**).

### EXAMPLE 4

### Synthesis and characterization of Tryptophol acetate and Tyrosol acetate

The inventors have isolated two molecules having masses of 180 and 203 from the K. marxianus crude extract, as was validated by LC-MS (**Fig 5**). Thereafter, the inventors have chemically synthesized both molecules, which were validated for weight and structural analysis using LC-MS and 1H NMR, respectively (**Figs**. **6** **and** **7**).

### EXAMPLE 5

### Effects of yogurt crude extracted molecules on QS systems

QS activation activity was calculated through normalization to the inducer bioluminescence signal alone. The crude extract comprising the 203 molecule activated QS in A. tumefaciens (**Fig. 8A**), and P. aeruginosa (**Fig. 8B**). In V. cholera, the crude extracted 203 molecule activated QS in low volume and inhibited QS in high volume (**Figs. 8C and 8D**). The 180 molecule activated QS in low volume and totally inhibited QS in high volume, in V. cholera. (**Fig. 9** ) with an IC₅₀ of 24.4 ± 2.4 µM.

### EXAMPLE 6

### Effects of probiotic yogurt (e.g., kefir) on fungal growth

The effect of the probiotic yogurt on fungi survival was further examined. Probiotic yogurt was supplemented to potato dextrose agar plates on which the fungus Sclerotinia sclerotiorum was cultured. S. sclerotiorum cultured on the probiotic yogurt-supplemented agar plates were shown to be inhibited for as long as 12 days (**Fig. 14A**) or 19 days (**Fig. 14B**). A similar inhibitory effect was observed for as long as 22 days post seeding in Botrytis (**Fig. 15**), and Penicillium (**Fig. 16**) yet to a lower extent.

### EXAMPLE 7

### Synthetic Tryptophol acetate molecule affects biofilm production

The inventors examined the effects of a synthetic Tryptophol acetate on the formation of biofilm by several types of bacteria. Tryptophol acetate did not seem to affect biofilm formation in P. aeruginosa when provided at 20 µM (**Fig. 11**). On the contrary, 50 µM of Tryptophol acetate had a significant influence on biofilm formation in both Salmonella (**Fig. 12**) and Staphylococcus aureus (**Fig. 13**).

### EXAMPLE 8

### Synergistic effect of Tyrosol acetate and Tryptophol acetate on QS in V. cholera

A synergistic effect of Tryptophol acetate and Tyrosol acetate on activation of QS in V. cholerae was observed (**Fig. 17**). The composition of Tryptophol acetate and Tyrosol acetate administered together at a ratio of 1:1, had an IC₅₀ of 11.6 ± 0.9 µM.

In addition, various combinations of Tryptophol acetate and Tyrosol acetate were examined in a mutated MM920 V. cholerae strain (ΔCqsA ΔluxQ). The biofilms produced by the mutated V. cholerae **(****Figs. 18A and 18E****)** were much thicker and denser than the respective biofilms generated upon addition of the autoinducer alone **(****Figs. 18B and 18F****;** which promoted quorum sensing, thus disrupted biofilm formation). Tryptophol acetate and Tyrosol acetate **(****Figs. 18D and 18H****),** and particularly the three compounds together **(****Figs. 18C and 18G****)** demonstrated a significant synergistic effect in disruption of normal biofilm growth - resulting in a different biofilm morphology.

### EXAMPLE 9

### Synthetic Tryptophol acetate reduces V. cholerae toxins load

The inventors then examined whether the synthetic Tryptophol acetate affects production levels of the V. cholerae toxin. Indeed, the synthetic Tryptophol acetate was found to reduce V. cholerae toxin levels in a dose dependent manner **(****Fig. 19****).**

### EXAMPLE 10

### Synthetic Tyrosol acetate molecule affects biofilm production

The inventors examined the effects of a synthetic Tyrosol acetate on Vibrio Cholera. Tyrosol acetate was found to inhibit V. cholerae biofilm and toxin productions. Further, Tyrosol acetate was shown affect QS-related genes' expression **(****Fig. 21****).**

### EXAMPLE 11

### Effects of probiotic yogurt (e.g., kefir) on bacterial growth and biofilm production

The effect of the probiotic yogurt on bacterial growth was further examined. The kefir crude extract interfered with QS pathways of all three bacterial strains examined. In case of the *vibrio cholerae MM920* mutant lacking the ability to synthesize its CAI-1 autoinducer, the kefir crude extract had a significant quorum sensing inhibitory (QSI) effect, as a direct correlation was apparent between kefir crude extract dilution and attenuation of bioluminescence reflecting the inhibition of QS in this strain **(****Fig. 22A****).** Further, it was shown that the kefir extract had a concentration-dependent QSI effect upon *Agrobacterium tumefaciens A136,* in which the QS pathway was induced by the 3-oxo-octanyl autoinducer **(****Fig. 22B****).** Interestingly, the results of *Vibrio harveyi MM30* bioluminescence assay utilizing the DPD autoinducer appeared to show that the kefir crude extract induced a quorum sensing activation (QSA) effect at all dilutions **(****Fig. 22C****).** As the bioluminescence assays attest that substances in the kefir extract affect QS pathways (inhibition or activation) of different bacteria **(****Figs. 22A-22C****),** the inventors further investigated whether the kefir extract influences formation of biofilm matrixes assembled by pathogenic bacteria. Indeed, it was revealed that the crude kefir extract significantly inhibited biofilms production of the prominent pathogenic bacteria *Pseudomonas aeruginosa, Salmonella enteritidis,* and *Staphylococcus aureus* **(****Fig. 22D****).** Specifically, the results showed that the biofilm volumes of each bacterial species, which were calculated through image analyses of the three-dimensional confocal microscopy images of the biofilm layers, were reduced by about 30% to 40% when the bacteria we co-incubated with the kefir extract, compared to untreated bacteria **(****Fig. 22D****).** Importantly, cell-viability assays confirmed that the kefir crude extract did not adversely impact bacterial cell proliferation and viability **(****Fig. 20****),** thus pointing to disruption of cell-cell communication as the likely factor contributing to reduction of biofilm development by the kefir.

### EXAMPLE 12

### Effect of Tryptophol acetate on Vibrio cholerae biofilm

The inventors examined the effect of tryptophol acetate on the biofilm of *V. cholerae* - the crucial component in its proliferation and pathogenicity, using MM920, a luminescent *V*. *cholerae* reporter strain lacking the oligonucleotide sequence encoding for the QS autoinducer CAI-1.

Biofilm matrixes of *V*. *cholerae VC1 WT* **(****Figs. 23Ai** and **23Aii****),** and the *V. cholerae MM920* mutant **(****Figs. 23Aiii** and **23Aiv****)** were grown for 24 hours with or without tryptophol acetate in the growth medium, and stained using the BacLight^{®} Dead/Live Kit to distinguish viable cells. The fluorescence microscopy analyses attested to the significant impact of tryptophol acetate upon biofilm morphology and organization, consistent with the anti-QS effect of the compound (e.g., **Fig.17B****).** Specifically, in case of *V*. *cholerae VC1 WT,* the control biofilm matrix (without addition of tryptophol acetate) appeared to be thin and non-uniform **(****Fig. 23Ai****);** this biofilm appearance is ascribed to the recently reports on the reciprocal relationship between functioning QS pathways in the *V*. *cholerae VC1* WT strain and assembly of the biofilm matrix by this bacterium. Further demonstrated is a significantly denser biofilm formed upon incubation of the proliferating bacteria with tryptophol acetate (100 µM, **Fig. 23Aii****),** which inhibited the important QS CAI-1 cascade. A similar dramatic effect of tryptophol acetate upon biofilm assembly was apparent in case of the *V. cholerae MM920* mutant **(****Figs. 23Aiii** and **23Av****).** The biofilm of the mutant bacteria alone appeared thick and dense **(****Fig. 23Aiii****)** due to lack of the CAI-1 QS cascade. Addition of the CAI-1 autoinducer to the bacterial growth medium re-introduced QS and concomitant disruption of biofilm integrity **(****Fig. 23Aiv****).** However, co-addition of tryptophol acetate and CAI-1 gave rise to re-formation of a dense and uniform biofilm layer **(****Fig. 23Av****),** reflecting inhibition of CAI QS pathway **(****Fig. 23Ai****).**

Biofilm volume analysis carried out through application of the quantitative crystal violet (CV) assay, depicted in **Fig. 23B****,** corroborates the fluorescence microscopy results in **Fig. 23A** , providing additional evidence for QS inhibition by tryptophol acetate. Indeed, given the fact that activation of QS at high cell density leads to reduced biofilm formation, the increased biofilm volume recorded upon addition of tryptophol acetate supports a direct inhibition of the QS pathways in *V*. *cholerae.*

To identify genes potentially regulated by tryptophol acetate, the inventors carried out a real time-quantitative PCR (RT-qPCR) analysis evaluating gene expression of *V*. *cholerae VC1 WT* at high cell density conditions **(****Fig. 23C****).** The RT-qPCR results provided dramatic evidence for the effect of tryptophol acetate in concentration of 100 µM upon gene expression pathways, specifically genes associated with QS, biofilm, and virulence regulation, accounting for the phenotypic modifications induced by the compound **(Figs. 23A-23B).** Specifically, the RT-qPCR data in reveal significant downregulation of *hapR* by tryptophol acetate **(****Fig. 23C****).** This result is notable, since *hapR* repression occurs in low cell density conditions; indeed, mimicking low cell density through *hapR* downregulation may account for the enhanced biofilm generation, observed upon incubation of *V*. *cholerae* with tryptophol acetate **(Figs. 23A-23B**This interpretation is corroborated by the remarkable upregulation of *vpsT* by tryptophol acetate **(****Fig. 23C****),** as expression of this gene, which is required for the biofilm formation, is enhanced in low cell density conditions. Furthermore, tryptophol acetate also resulted in a lower expression of *hapA* **(****Fig. 23C****),** a downstream gene regulated by *hapR.* Repression of *hapA* may in fact point to potential therapeutic benefits of the herein disclosed probiotic yogurt (e.g., kefir) as hapA is associated with varied pathogenic effects induced by *V. cholera,* such as fluid generation and diarrhea. Importantly, mimicking low cell density for *V*. *cholerae* by tryptophol acetate is expected to promote genetic cascades leading to enhanced virulence. Indeed, the RT-qPCR experiment demonstrated upregulation of *aphA,* associated with *V*. *cholerae* toxin production **(****Fig. 23C****).** Surprisingly, however, the RT-qPCR analysis reveals that *ctxA,* a virulence-inducing gene that is downstream in the *aphA*-induced virulence cascade, was in fact significantly repressed by tryptophol acetate **(****Fig. 23C****).** To assess the phenotypic aspects of this result, the inventors measured and showed that Tyrosol acetate poses reduction of toxin production by *V. cholerae* in a dose dependent manner **(****Fig. 19****).**

### EXAMPLE 13

### Probiotic yogurt (e.g., kefir) in the treatment of inflammatory bowel disease

### Experiment Outline:

Study Duration (each cycle): 10 days; Group size: n = 4 or 6; Number of groups =10; Animals: male black mice, 6-8 weeks old at study initiation; Model: Colitis/Crohn's disease induced in mice by 2.5% (w/v) Dextran Sodium Sulfate (DSS) supplementation to the drinking water. DSS-water was replaced every day for 7 days.

### Treatment:

The test items: probiotic yogurt, or a mixture of both molecules (Tyrosol acetate and tryptophol acetate) in final concentration of either 25 µM (repetition 2) or 50 µM (repetition 3) were delivered in oral route (gavage) as described below, once daily from day 1 until day 7.

**Table 1 - Test items examination outline in a murine IBD model**

| **Group Number** | **Group Name** | **Description** | **DSS** |
|---|---|---|---|
| **1** | Control +W | Regular food-no DSS | - |
| **2** | DSS + W | Regular food | + |
| **3** | Control + Y | Testfood (Yogurt)-no DSS | - |
| **4** | DSS+ Y | Testfood (Yogurt) | + |
| **5** | Control + mole | Molecular Test Item (oral) | - |
| **6** | DSS + mole | Molecular Test Item (oral) | + |
| **7** | Control + Yc | Testfood (Commercial yogurt)-no DSS | - |
| **8** | DSS + Yc | Testfood (Commercial yogurt) | + |
| **9** | Y + DSS + Y | Early administration of yogurt before DSS and after | + |
| **10** | Yc + DSS + Yc | Early administration of commercial yogurt before DSS and after | + |

### Examinations throughout the experiment:

Morbidity and mortality - once a day; Clinical signs - every other day; Body weight - on the first day and every other day thereafter; Rectal bleeding and stool consistency scoring (as indicated in Table 2 below) - on the first day (day 0, or baseline) and on day 7.

### Termination:

Bleeding for cytokines; Clinical Scoring (according to Table 2); Animals were sacrificed on day 10, and colons were excised, measured, and weighed; Colon fixed in 10% buffered formalin and the distal colon anatomical region was paraffin-embedded for histology. The histological samples were staining with Hematoxylin & Eosin for histopathology analysis and scoring based on loss of crypt cells from the gut lining and infiltration of inflammatory cells into the colon.

**Table 2 - Clinical score**

| **Score (Points)** | **Stool consistency** | **Rectal bleeding** |
|---|---|---|
| **0** | Normal | Negative |
| **1** | Loose stool | Negative |
| **2** | Loose stool | Occult |
| **3** | Diarrhea | Occult |
| **4** | Diarrhea | Positive |

The inventors showed that the herein disclosed probiotic yogurt and molecules identified therein reduce weight loss in a murine inflammatory bowel disease (IBD) model. Significantly reduced body weight loss were observed in mice treated with Y+DSS+Y; DSS+Y and DSS+Mole (either at 25 µM, or 50 µM) compared to DSS+W **(****Fig. 24****).** In addition, there was no significant body weight loss effect in the group receiving commercial yogurt (Yc+DSS+Yc or DSS+Yc).

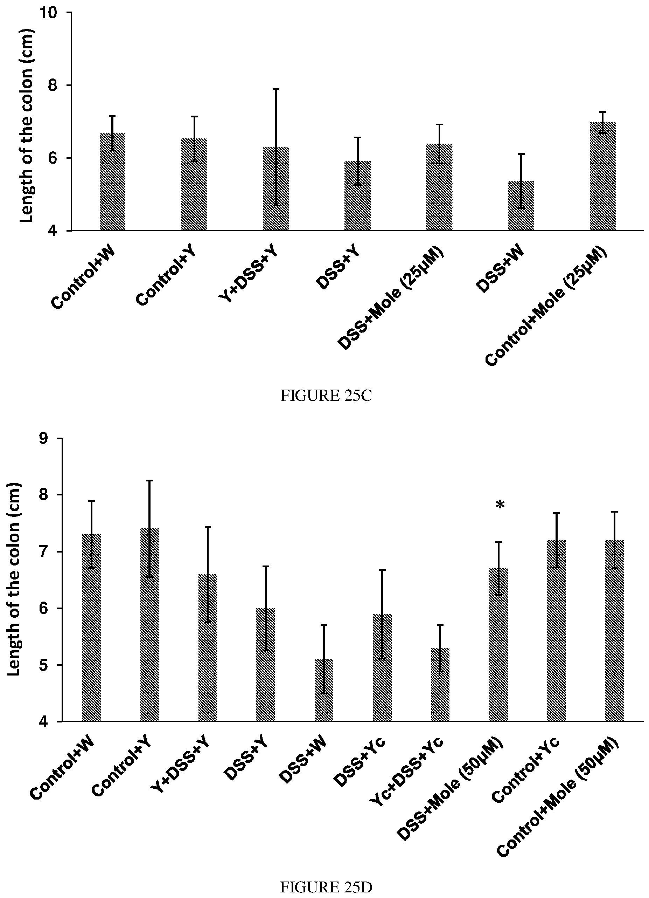

The inventor showed that the herein disclosed probiotic yogurt and molecules identified therein reduce colon shortening in a murine IBD model **(****Fig. 25****).** Colon length were measured from the rectum to the caecum at the endpoint of the experiment. Shortening of the colon was observed in mice treated with DSS (DSS+W) compared the colon of mice treated with DSS and fed with the herein disclosed probiotic yogurt before the DSS (Y+DSS+Y). Furthermore, a statistically significant reduced shortening of colon length was observed in mice treated with molecules (50 µM) identified in the herein disclosed probiotic yogurt.

The inventors further examined the effect of the herein disclosed probiotic yogurt and of molecules identified therein on disease activity index (DAI). The results showed a significant decrease in the clinical score in mice treated with either yogurt before DSS (Y+DSS+Y; **Fig. 26A****)** or molecules identified in the herein disclosed probiotic yogurt in concentration of 50 µM (DSS + Molec; **Fig. 26B****).**

Colon histopathological sections revealed intensive tissue damage with loss of crypt cells from the gut lining and infiltration of inflammatory cells into the colon in all DSS-treated mice **(****Fig. 27A****).** In colon of mice fed with yogurt before DSS (Y+DSS+Y) tissue structure was preserved with only minimal inflammation and initial signs of colon tissue repair (encircled; **Fig. 27B****).**

### EXAMPLE 14

### Administration of probiotic yogurt (e.g., kefir) in the treatment of cutaneous ulcers

The inventors showed that Leishmaniosis ulcers can be treated using the probiotic yogurt disclosed herein. Briefly, healing was observed after application of the yogurt mixture, wherein the application followed a three-month conventional treatment which failed to assist the patient. The conventional treatment included PENTOSTAM injection after local anesthesia with ESRACAIN injection. In addition, due to the thick scribble, the patient smeared SALIKAREN. In the yogurt treatment, the patient applied the probiotic yogurt over all of his ulcers, and bandaged the ulcers twice a day (morning and evening). The results showed accelerated healing of cutaneous leishmaniosis after 11-14 days of treatment **(****Fig. 28****).**

Further, in a case where sutures were required for the fusion of an incision, which were not performed because more than 24 hours have passed since the injury, the probiotic yogurt was applied, and wound healing was evident as early as four days after initial application **(****Fig. 29****).**

### EXAMPLE 15

### Tryptophol acetate and Tyrosol acetate anti-inflammatory activity

The inventors showed that the production of both proinflammatory cytokines, IL-1α and IL-6, were dramatically reduced in macrophages which were cultured in presence of tryptophol acetate, tyrosol acetate, or both **(****Fig. 30****).** The other 4-Ethyl Phenol derivatives, dopamine HCl, and caffeic acid, showed comparable results.

### EXAMPLE 16

### Tryptophol acetate anti-neurodegenerative activity

The inventors examined the activity of a composition comprising tryptophol acetate in a common in vitro neurodegenerative model. Both a mixture of molecules extracted from the probiotic yogurt disclosed herein, and tryptophol acetate, were shown to effectively reduced amyloid beta fibrillation **(****Fig. 31****)** after 12 hours of incubation. Further, inhibition of amyloid beta by tryptophol acetate, was shown to be in a dose dependent manner **(****Fig. 32****).**

The inventors further examined the effect of either a crude extract of the probiotic yogurt disclosed herein or tryptophol acetate on amyloid beta fibrillation after an incubation period of 24 hr. In the presence of crude extract of the probiotic yogurt disclosed herein, fibrillation formation was completely inhibited **(****Fig. 33B****).** Fibrillation was also significantly inhibited in the presence of tryptophol acetate, in a dose dependent manner **(****Figs. 33C-33D****).**

## Claims

1. A composition comprising:
a Tryptophol derivative, which is Tryptophol acetate,
a 4-Ethyl-Phenol derivative, which is Tyrosol acetate, and
at least one pharmaceutically acceptable carrier,
wherein said Tryptophol derivative and said 4-Ethyl-Phenol derivative are in a ratio of 10:1 - 1:10 w/w, and
wherein the compounds are biosynthesizable using a cell culture comprising *Kluyveromyces marxianus.*

2. The composition of claim 1, wherein said Tryptophol derivative and 4-Ethyl-Phenol derivative are each present at a concentration of at least 1 µM within said composition.

3. The composition of claim 1, wherein said Tryptophol derivative is at a concentration of at least 0.1 µM within said composition.

4. The composition of any of claims 1 to 3, wherein said Tryptophol derivative and said 4-Ethyl-Phenol derivative are in w/w ratio ranging from 2:1 (w/w) - 1:2 (w/w).

5. The composition of any of claims 1 to 4, further comprising *Kluyveromyces marxianus,* and at least one probiotic microorganism.

6. The composition of any one of claims 1 to 5, for use in reducing pathogenic microbial activity in a subject, or for use in treating an infectious disease, an inflammatory disease, or an amyloid aggregates-related disease in a subject.

7. The composition for use according to claim 6, wherein said infectious disease comprises a load of a microorganism, a biofilm derived therefrom, or both.

8. The composition for use according to claim 7, wherein said microorganism is a virus, a fungus, a parasite, a yeast, a bacterium, or a protozoon.

9. The composition for use according to claim 8, wherein said fungus belongs to the genus Botrytis, Penicillium or Sclerotinia.

10. The composition for use according to claim 8, wherein said bacterium belongs to the genus Vibrio, Salmonella, Staphylococcus, or Pseudomonas.

11. The composition for use according to any of claims 6 to 10, having a half maximal inhibitory concentration (IC₅₀) for each of said Tryptophol derivative and said 4-Ethyl-Phenol derivative of 1-1,000 µM.

12. The composition for use according to any of claims 6 to 11, wherein:
a. said inflammatory disease is an inflammatory bowel disease, optionally ulcerative colitis or Crohn's disease; or
b. said amyloid aggregates-related disease is a neurodegenerative disease.

## Patentansprüche

1. Zusammensetzung, umfassend:
ein Tryptopholderivat, bei dem es sich um Tryptopholacetat handelt,
ein 4-Ethylphenolderivat, bei dem es sich um Tyrosolacetat handelt, und
mindestens einen pharmazeutisch akzeptablen Träger,
wobei das Tryptopholderivat und das 4-Ethylphenolderivat in einem Verhältnis von 10:1 bis 1:10 (Gew./Gew.) vorliegen, und
wobei die Verbindungen unter Verwendung einer Zellkultur, die Kluyveromyces marxianus umfasst, biosynthetisierbar sind.

2. Zusammensetzung nach Anspruch 1, wobei das Tryptopholderivat und das 4-Ethylphenolderivat jeweils in einer Konzentration von mindestens 1 µM in der Zusammensetzung vorliegen.

3. Zusammensetzung nach Anspruch 1, wobei das Tryptopholderivat in einer Konzentration von mindestens 0,1 µM in der Zusammensetzung vorliegt.

4. Zusammensetzung nach einem der Ansprüche 1 bis 3, wobei das Tryptopholderivat und das 4-Ethylphenolderivat in einem Gewichtsverhältnis von 2:1 (Gew./Gew.) bis 1:2 (Gew./Gew.) vorliegen.

5. Zusammensetzung nach einem der Ansprüche 1 bis 4, ferner umfassend Kluyveromyces marxianus und mindestens einen probiotischen Mikroorganismus.

6. Zusammensetzung nach einem der Ansprüche 1 bis 5 zur Verwendung beim Verringern von pathogener mikrobieller Aktivität in einem Subjekt oder zur Verwendung beim Behandeln einer Infektionskrankheit, einer Entzündungskrankheit oder einer Amyloidaggregat-bezogenen Krankheit in einem Subjekt.

7. Zusammensetzung zur Verwendung nach Anspruch 6, wobei die Infektionskrankheit eine Belastung durch einen Mikroorganismus, einen davon abgeleiteten Biofilm oder beides umfasst.

8. Zusammensetzung zur Verwendung nach Anspruch 7, wobei der Mikroorganismus ein Virus, ein Pilz, ein Parasit, eine Hefe, ein Bakterium oder ein Protozoon ist.

9. Zusammensetzung zur Verwendung nach Anspruch 8, wobei der Pilz zur Gattung Botrytis, Penicillium oder Sclerotinia gehört.

10. Zusammensetzung zur Verwendung nach Anspruch 8, wobei das Bakterium zur Gattung Vibrio, Salmonella, Staphylococcus oder Pseudomonas gehört.

11. Zusammensetzung zur Verwendung nach einem der Ansprüche 6 bis 10, wobei die halbe maximale Hemmkonzentration (IC₅₀) für jedes von dem Tryptopholderivat und dem 4-Ethylphenolderivat 1 bis 1.000 µM beträgt.

12. Zusammensetzung zur Verwendung nach einem der Ansprüche 6 bis 11, wobei:
a. die Entzündungskrankheit eine entzündliche Darmerkrankung ist, optional Colitis ulcerosa oder Morbus Crohn; oder
b. die Amyloidaggregat-bezogene Erkrankung eine neurodegenerative Erkrankung ist.

## Revendications

1. Composition, comprenant :
un dérivé du tryptophol, qui est l'acétate de tryptophol,
un dérivé du 4-éthylphénol, qui est l'acétate de tyrosol, et
au moins un excipient pharmaceutiquement acceptable,
dans laquelle ledit dérivé du tryptophol et ledit dérivé du 4-éthylphénol sont dans un rapport de 10:1 à 1:10 en poids/poids (p/p), et
dans laquelle les composés sont biosynthétisables à l'aide d'une culture cellulaire comprenant *Kluyveromyces marxianus.*

2. Composition de la revendication 1, dans laquelle ledit dérivé du tryptophol et ledit dérivé du 4-éthylphénol sont chacun présents à une concentration d'au moins 1 µM dans ladite composition.

3. Composition de la revendication 1, dans laquelle ledit dérivé du tryptophol est présent à une concentration d'au moins 0,1 µM dans ladite composition.

4. Composition de l'une quelconque des revendications 1 à 3, dans laquelle ledit dérivé du tryptophol et ledit dérivé du 4-éthylphénol sont dans un rapport en p/p compris entre 2:1 (p/p) et 1:2 (p/p).

5. Composition de l'une quelconque des revendications 1 à 4, comprenant en outre *Kluyveromyces marxianus* et au moins un micro-organisme probiotique.

6. Composition de l'une quelconque des revendications 1 à 5, pour une utilisation dans la réduction de l'activité microbienne pathogène chez un sujet, ou pour une utilisation dans le traitement d'une maladie infectieuse, d'une maladie inflammatoire ou d'une maladie liée aux agrégats amyloïdes chez un sujet.

7. Composition pour une utilisation selon la revendication 6, dans laquelle ladite maladie infectieuse comprend une charge d'un micro-organisme, un biofilm dérivé de celui-ci, ou les deux.

8. Composition pour une utilisation selon la revendication 7, dans laquelle ledit micro-organisme est un virus, un champignon, un parasite, une levure, une bactérie ou un protozoaire.

9. Composition pour une utilisation selon la revendication 8, dans laquelle ledit champignon appartient au genre Botrytis, Penicillium ou Sclerotinia.

10. Composition pour une utilisation selon la revendication 8, dans laquelle ladite bactérie appartient au genre Vibrio, Salmonella, Staphylococcus ou Pseudomonas.

11. Composition pour une utilisation selon l'une quelconque des revendications 6 à 10, ayant une concentration inhibitrice médiane (IC₅₀), pour chacun dudit dérivé du tryptophol et dudit dérivé du 4-éthylphénol, de 1 à 1 000 µM.

12. Composition pour une utilisation selon l'une quelconque des revendications 6 à 11, dans laquelle :
a. ladite maladie inflammatoire est une maladie inflammatoire de l'intestin, éventuellement une colite ulcéreuse ou la maladie de Crohn ; ou
b. ladite maladie liée aux agrégats amyloïdes est une maladie neurodégénérative.
